# EUROPEAN PATENT APPLICATION

(11) **EP 4 282 331 A1**
(43) Date of publication of application: **29.11.2023**
(21) Application number: 23175364.1
(22) Date of filing: 25.05.2023
(51) Int. Cl.: A61B 5/145

(54) **GLUCOSE SENSOR**

(30) Priority: 27.05.2022 US 202263365451 P; 12.05.2023 US 202318316708
(71) Applicant: Medtronic MiniMed, Inc., Northridge, CA 91325 (US)
(72) Inventor: Garai, Ellis, Northridge, 91325 (US); Tsang, Melissa, Northridge, 91325 (US); Srinivasan, Akhil, Northridge, 91325 (US); Petkus, Bradley, Northridge, 91325 (US)
(74) Representative: Zimmermann & Partner Patentanwälte mbB

(57) **Abstract**

An example device includes an electrochemical cell configured to generate a first electrical signal indicative of an amount of glucose in a fluid of a person, the electrochemical cell comprising a working electrode, a counter electrode, and a reference electrode; and a background electrode configured to not catalyze a reaction with glucose and is configured to generate a second electrical signal indicative of an amount of electrochemical interference proximate the electrochemical cell.

## Description

### TECHNICAL FIELD

This application claims the benefit of U.S. Provisional Application No. 63/365,451, filed May 27, 2022, and entitled, "GLUCOSE SENSOR," the entire content of which is incorporated herein by reference.

### TECHNICAL FIELD

This disclosure relates to glucose sensors.

### BACKGROUND

Glucose sensors are configured to detect and/or quantify the amount of glucose in a patient's body (e.g., interstitial glucose or possibly blood glucose), which enables patients and medical personnel to monitor physiological conditions within the patient's body. In some examples, it may be beneficial to monitor glucose levels on a continuing basis (e.g., in a diabetic patient). Thus, glucose sensors have been developed for use in obtaining an indication of glucose levels in a diabetic patient. Such indications are useful in monitoring and/or adjusting a treatment regimen, which typically includes administration of insulin to the patient.

A patient can measure their blood glucose (BG) using a BG measurement device (i.e., glucose meter), such as a test strip meter. A continuous glucose measurement system (or a continuous glucose monitor (CGM)) may be configured to determine interstitial glucose; although possible for CGM to determine blood glucose. A hospital hemocue may also be used to determine glucose level. CGMs may be beneficial for patients who desire to take more frequent glucose measurements. Some example CGM systems include subcutaneous (or short-term) sensors and implantable (or long-term) sensors. A CGM system may execute an initialization sequence when the CGM is inserted into a patient. The initialization sequence may speed up sensor equilibration and may allow a CGM system to provide reliable glucose measurements earlier.

### SUMMARY

In general, this disclosure describes systems and techniques for determining electrochemical interference of glucose sensors (e.g., a continuous glucose monitor or "CGM") based on background signal detection. More particularly, this disclosure describes systems and devices including one or more background electrodes configured to generate a signal corresponding to electrochemical interference. The electrochemical interference may interfere with accurately determining the glucose level. By determining the signal corresponding to the electrochemical interference, a device (e.g., glucose sensor or possibly some other device) may more accurately determine the glucose level.

In one example, this disclosure describes a device including an electrochemical cell configured to generate a first electrical signal indicative of an amount of glucose in a fluid of a person, the electrochemical cell comprising a working electrode, a counter electrode, and a reference electrode; and a background electrode configured to not catalyze a reaction with glucose and is configured to generate a second electrical signal indicative of an amount of electrochemical interference proximate the electrochemical cell.

In another example, this disclosure describes a glucose monitor system including an electrochemical cell configured to generate a first electrical signal indicative of an amount of glucose in a fluid of a person, the electrochemical cell disposed on a first side of a first insulative substrate; a background electrode configured to not catalyze a reaction with glucose and is configured to generate a second electrical signal indicative of an amount of electrochemical interference, the background electrode disposed on a first side of a second insulative substrate, wherein a second side opposite the first side of the first insulative substrate is adjacent to a second side opposite the first side of the second insulative substrate; and an electrical circuit configured to receive the first electrical signal indicative of the amount of glucose and the second electrical signal indicative of the amount of electrochemical interference, wherein the electrical circuit is configured to determine an amount of glucose based on the first signal and the second signal..

In another example, this disclosure describes a method including receiving, from an electrochemical cell, a first signal indicative of an amount of glucose in a fluid of a person; receiving, from a background electrode configured to not catalyze a reaction with glucose and positioned proximate the electrochemical cell, a second signal indicative of an amount of electrochemical interference; and determining a glucose level of the fluid of the person based on the first and second signals.

Further disclosed herein is an example device which includes an electrochemical cell configured to generate a first electrical signal indicative of an amount of glucose in a fluid of a person, the electrochemical cell comprising a working electrode, a counter electrode, and a reference electrode; and a background electrode configured to not catalyze a reaction with glucose and is configured to generate a second electrical signal indicative of an amount of electrochemical interference proximate the electrochemical cell.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a block diagram illustrating an example glucose sensor, in accordance with one or more examples described in this disclosure.
FIG. 2 is a block diagram illustrating example sensor electrodes and a voltage being applied to the sensor electrodes.
FIG. 3 is a cross-sectional view of an example medical device including a glucose sensor.
FIG. 4 is a top view of an example glucose sensor.
FIG. 5 is a top view of an example drug eluting device.
FIG. 6 is an exploded view of the example medical device including the glucose sensor of FIG. 3 and the example background sensor of FIG. 5.
FIG. 7 is a perspective view of an example circuit board of a medical device for using with a glucose sensor.
FIG. 8A is a top view of a distal portion of an example glucose sensor.
FIG. 8B is a cross-sectional view of the distal portion of the example glucose sensor of FIG. 8A.
FIG. 9A is a top view of a distal portion of an example drug eluting device including a background electrode.
FIG. 9B is a cross-sectional view of the distal portion of the example drug eluting device of FIG. 9A.
FIG. 9C is a top view of a distal portion of another example drug eluting device including a background electrode.
FIG. 9D is a cross-sectional view of the distal portion of the example drug eluting device of FIG. 9C.
FIG. 10A is a top view of a distal portion of another example drug eluting device.
FIG. 10B is a cross-sectional view of the distal portion of FIG. 10A.
FIG. 11A is a top view of the example glucose sensor of FIG. 8A.
FIG. 11B is a top view of the example drug eluting device of FIG. 10A arranged for visual comparison with the example glucose sensor of FIG. 11A.
FIG. 12A is a top view of a distal portion of another example drug eluting device including another example background electrode.
FIG. 12B is a cross-sectional view of the distal portion of the example drug eluting device and the example background electrode of FIG. 12A.
FIG. 13A is a top view of a distal portion of another example drug eluting device including another example background electrode.
FIG. 13B is a cross-sectional view of the distal portion of the example drug eluting device and the example background electrode of FIG. 13A.
FIG. 14A is a top view of a distal portion of another example drug eluting device including another example background electrode.
FIG. 14B is a cross-sectional view of the distal portion of the example drug eluting device and the example background electrode of FIG. 14A.
FIG. 15A is a top view of a distal portion of another example drug eluting device including another example background electrode.
FIG. 15B is a cross-sectional view of the distal portion of the example drug eluting device and the example background electrode of FIG. 15A.
FIG. 16A is a top view of a distal portion of another example drug eluting device including another example background electrode.
FIG. 16B is a bottom view of the distal portion of the example drug eluting device of FIG. 16A.
FIG. 16C is a cross-sectional view of the distal portion of the example drug eluting device and the example background electrode of FIG. 16A.
FIG. 17A is a top view of a distal portion of another example drug eluting device including another example background electrode.
FIG. 17B is a bottom view of the distal portion of the example drug eluting device of FIG. 16A.
FIG. 17C is a cross-sectional view of the distal portion of the example drug eluting device and the example background electrode of FIG. 16A.
FIG. 18A is a top view of a distal portion of another example drug eluting device including another example background electrode.
FIG. 18B is a bottom view of the distal portion of the example drug eluting device of FIG. 16A.
FIG. 18C is a cross-sectional view of the distal portion of the example drug eluting device and the example background electrode of FIG. 16A.
FIG. 19A is a top view of a distal portion of another example drug eluting device including another example background electrode.
FIG. 19B is a bottom view of the distal portion of the example drug eluting device of FIG. 16A.
FIG. 19C is a cross-sectional view of the distal portion of the example drug eluting device and the example background electrode of FIG. 16A.
FIG. 20A is a top view of a distal portion of another example drug eluting device including another example background electrode.
FIG. 20B is a bottom view of the distal portion of the example drug eluting device of FIG. 16A.
FIG. 20C is a cross-sectional view of the distal portion of the example drug eluting device and the example background electrode of FIG. 16A.
FIG. 21A is a top view of a distal portion of another example drug eluting device including another example background electrode.
FIG. 21B is a cross-sectional view of the distal portion of the example drug eluting device and the example background electrode of FIG. 21A.
FIG. 22A is a top view of a distal portion of another example drug eluting device including another example background electrode.
FIG. 22B is a cross-sectional view of the distal portion of the example drug eluting device and the example background electrode of FIG. 22A.
FIG. 23 is a top view of another example glucose sensor.
FIG. 24 is a top view of another example glucose sensor.
FIG. 25 is a top view of another example glucose sensor.
FIG. 26A is a top view of a distal portion of another example glucose sensor including an example background electrode.
FIG. 26B is a bottom view of the distal portion of the example glucose sensor of FIG. 26A.
FIG. 26C is a cross-sectional view of the distal portion of the example glucose sensor and the example background electrode of FIG. 26A.
FIG. 27A is a top view of a distal portion of another example glucose sensor including an example background electrode.
FIG. 27B is a bottom view of the distal portion of the example glucose sensor of FIG. 26A.
FIG. 27C is a cross-sectional view of the distal portion of the example glucose sensor and the example background electrode of FIG. 26A.
FIG. 28A is a top view of a distal portion of another example drug eluting device without a background electrode.
FIG. 28B is a cross-sectional view of the distal portion of the example drug eluting device of FIG. 28A.
FIG. 29 is a flowchart illustrating an example method of determining a glucose level of a fluid of a person.

### DETAILED DESCRIPTION

Electrochemical interference can cause the CGM to provide an inaccurate glucose reading, which may lead to imprecise diagnoses if the readings are used to compute insulin dosage. A glucose sensor may include an electrochemical cell which may be used to determine a glucose level of a patient. As one example, the glucose sensor may determine interstitial glucose level. The electrochemical cell may apply electrical energy via one or more electrodes (e.g., working electrodes and counter electrodes) displaced within a fluid and/or tissue of a patient, and based on the impedance, determine the glucose level. The electrochemical cell may also include a reference electrode for setting a reference voltage, but in some examples, the reference electrode may not be needed or may be part of a counter electrode.

The electrochemical cell may be susceptible to electrochemical interference. Electrochemical interference may be caused by one or more interferants, the one or more interferants are capable of being sensed by the electrochemical cell and causing the electrochemical cell to output a signal. In other words, an interferant may cause the electrochemical cell to output a signal in the same manner as glucose, and may effectively masquerade as glucose. Electrochemical interference may cause uncertainty in a glucose level based on the signal generated by the electrochemical cell. Acetaminophen is one example of an electrochemical interferant.

In accordance with the techniques of the disclosure, devices, systems, and techniques for detecting and compensating for electrochemical interference include a background electrode configured to catalyze a reaction with glucose and to generate a signal indicative of an amount of electrochemical interference. In some examples, the background electrode may be distinct from the example electrodes of the electrochemical cell. For instance, unlike working electrodes of the electrochemical cell, the background electrode may be configured to not catalyze a reaction with glucose. Also, a counter electrode of the electrochemical cell may not generate a signal, but is part of the current loop used to determine the glucose level. Unlike the counter electrode, the background electrode may be configured to generate an electrical signal indicative of an amount of electrochemical interference proximate the electrochemical cell.

The background electrode is configured to be positioned proximate the electrochemical cell. In some examples, the background electrode may be included on a separate device, such as a drug eluting device configured to be proximate a glucose sensor and introduced into a patient along with the glucose sensor. In other examples, the background electrode is included with the glucose sensor.

The signal generated by the background electrode may be subtracted from a signal generated by an electrochemical cell. For example, the electrochemical cell may be configured to catalyze a reaction with glucose and generate a signal indicative of an amount of glucose in response to, or caused by, the reaction. The electrochemical cell may additionally generate an additional signal caused by electrochemical interference, e.g., the signal generated by one or more "interferant" materials that may react via an electrocatalytic reaction with the counter and/or working electrodes. The additional signal due to electrochemical interference may be generated along with the signal generated in response to glucose, and the electrochemical cell may output the generated signals as a combined signal. The signal generated by the background electrode, without generating a signal in response to glucose, may be caused by the same or similar electrochemical interference, e.g., the background electrode may be positioned proximate the electrochemical cell and may be exposed to the same amount and/or concentration of "interferants". The signal generated by the background electrode may then be used to compensate for and/or remove the portion of the signal generated by the electrochemical cell that is due to electrochemical interference. The amount of glucose may then be determined based on both the signal generated by the electrochemical cell (a portion of which is due to electrochemical interference) and the signal generated by the background electrode (used to compensate/correct the signal generated by the electrochemical cell).

In some examples, the signal generated by the background electrode may be directly subtracted from the signal generated by the electrochemical cell, e.g., directly from iSig from the working electrode described below. In some examples, the signal generated by the background electrode may be scaled by a constant and subtracted from the signal (e.g., iSig) generated by the electrochemical cell. In some examples, the signal generated by the background electrode may be scaled based on one or more factors. The one or more factors may include, but are not limited to, a time period over which the signal is generated by the electrochemical cell (e.g., a day of wear of the CGM), electrochemical impedance spectroscopy (EIS) signals, a time of day, a rate of change of the signal generated by the electrochemical cell (e.g., iSig), a rate of change of signal generated by the background electrode, and the like.

In some examples, a when a reference electrode is present in the electrochemical cell, a constant voltage may be applied. The reference electrode may support a working electrode and background electrode with a shared counter electrode.

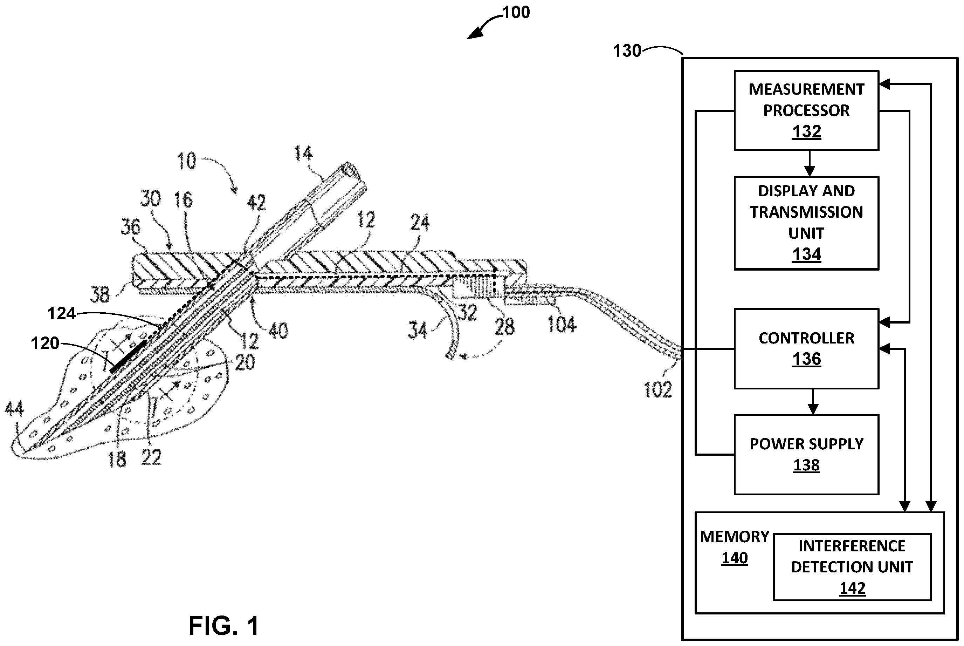

FIG. 1 is a block diagram illustrating glucose level monitoring device 100 in accordance with one or more examples described in this disclosure. Monitoring device 100 may include a sensor that is inserted under the skin of a patient (e.g., in vivo), such as near the stomach of the patient or in the arm of the patient (e.g., subcutaneous connection). The sensor of monitoring device 100 may be configured to measure the interstitial glucose level, which is the glucose found in the fluid between the cells of the patient. Monitoring device 100 may be configured to continuously or periodically sample the glucose level and rate of change of the glucose level over time.

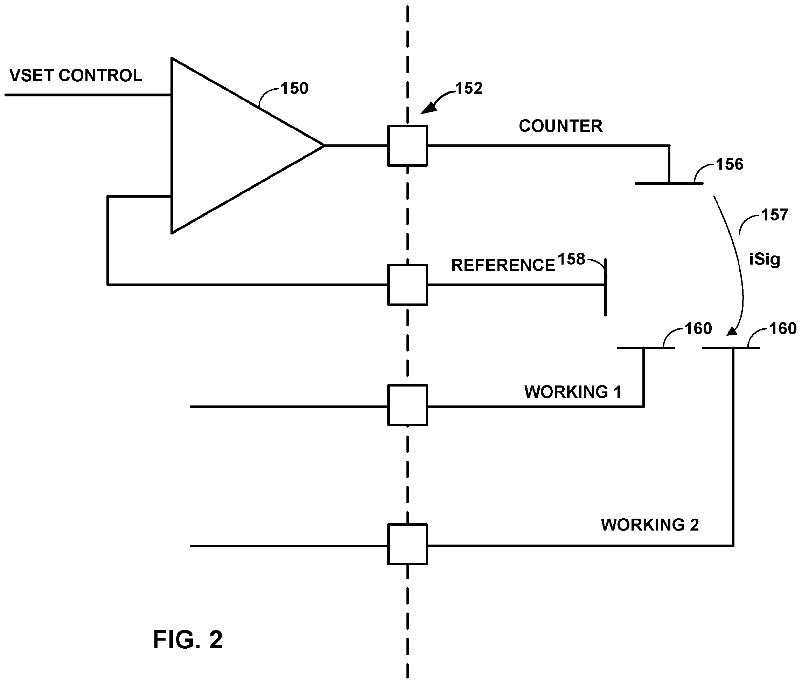

In particular, FIG. 1 is a perspective view of a subcutaneous sensor insertion set and a block diagram of sensor electronics device 130 of monitoring device 100 according to an example of the disclosure. As illustrated in FIG. 1, subcutaneous sensor set 10 is provided for subcutaneous placement of an active portion of flexible glucose sensor 12 at a selected site in the body of a patient. The subcutaneous or percutaneous portion of sensor set 10 includes a hollow, slotted insertion needle 14, and cannula 16. Needle 14 is used to facilitate quick and easy subcutaneous placement of cannula 16 at the subcutaneous insertion site. Inside cannula 16 is electrochemical cell 18 (e.g., a glucose sensing portion) of glucose sensor 12, which is configured to expose one or more glucose sensor electrodes 20 to the bodily fluids (e.g., blood or interstitial fluid) of the patient through window 22 formed in cannula 16. In one example, one or more glucose sensor electrodes 20 may include a counter electrode 156, a reference electrode 158, and one or more working electrodes 160. Examples of the counter electrode 156, reference electrode 158, and working electrode(s) 160 are described in more detail with respect to FIG. 2. After insertion, insertion needle 14 is withdrawn to leave cannula 16 with electrochemical cell 18 and glucose sensor electrodes 20 in place at the selected insertion site.

In some examples, subcutaneous sensor set 10 facilitates accurate placement of flexible thin film electrochemical glucose sensor 12 of the type used for monitoring specific blood parameters representative of a condition of the patient. Glucose sensor 12 monitors glucose levels in the body, and may be used in conjunction with automated or semi-automated medication infusion pumps of the external or implantable type (not shown) to control delivery of insulin to the patient.

Examples of flexible electrochemical glucose sensor 12 are constructed in accordance with thin film mask techniques to include elongated thin film conductors embedded or encased between layers of a selected insulative material such as polyimide film or sheet, and membranes. Glucose sensor electrodes 20 at a tip, e.g., distal end of electrochemical cell 18 are exposed through one of the insulative layers for direct contact with patient blood or other body fluids, when electrochemical cell 18 (or active portion) of glucose sensor 12 is subcutaneously placed at an insertion site. Electrochemical cell 18 is joined to connection portion 24 that terminates in conductive contact pads, or the like, which are also exposed through one of the insulative layers. In other examples, other types of implantable sensors, such as chemical based, optical based, or the like, may be used.

Connection portion 24 and the contact pads are generally adapted for a direct wired electrical connection to a suitable monitor or sensor electronics device 130 for monitoring a condition of the patient in response to signals derived from glucose sensor electrodes 20. In some examples, connection portion 24 and the contact pads are adapted for electrical connection via physical contact, e.g., a pressure contact between connection portion 24 and the contact pads. Connection portion 24 may be conveniently connected electrically to the monitor or sensor electronics device 130 or by connector block 28. Thus, in accordance with examples of the disclosure, subcutaneous sensor sets 10 may be configured or formed to work with either a wired or a wireless characteristic monitor system.

Glucose sensor electrodes 20 may be used in a variety of sensing applications and may be configured in a variety of ways. For example, glucose sensor electrodes 20 may be used in physiological parameter sensing applications in which some type of biomolecule is used as a catalytic agent. For example, glucose sensor electrodes 20 may be used in a glucose and oxygen sensor having a glucose oxidase (GOx) enzyme catalyzing a reaction with glucose sensor electrodes 20. Glucose sensor electrodes 20, along with a biomolecule or some other catalytic agent, may be placed in a human body in a vascular or non-vascular environment. For example, glucose sensor electrodes 20 and biomolecules may be placed in a vein and be subjected to a blood stream, or may be placed in a subcutaneous or peritoneal region of the human body.

Sensor electronics device 130 may include measurement processor 132, display and transmission unit 134, controller 136, power supply 138, and memory 140. Sensor electronics device 130 may be coupled to the sensor set 10 by cable 102 through a connector that is electrically coupled to connector block 28 of connection portion 24. In other examples, the cable may be omitted and sensor electronics device 130 may include an appropriate connector for direct connection to connection portion 104 of sensor set 10. Sensor set 10 may be modified to have connector portion 104 positioned at a different location, e.g., on top of the sensor set to facilitate placement of sensor electronics device 130 over the sensor set.

In some examples, measurement processor 132, display and transmission unit 134, and controller 136 may be formed as separate semiconductor chips. However, other examples may combine measurement processor 132, display and transmission unit 134, and controller 136 into a single or multiple customized semiconductor chips. In general, measurement processor 132 may be configured to receive a current, voltage, and/or impedance from glucose sensor electrodes 20. Glucose sensor electrodes 20 may generate a sensor signal indicative of a concentration of a physiological characteristic being measured. For example, the sensor signal may be indicative of a glucose reading. The sensor signal may be measured at a working electrode 160 (illustrated in FIG. 2) of glucose sensor electrodes 20. In an example of the disclosure, the sensor signal may be a current (e.g., iSig) measured at the working electrode 160 that flows from working electrode 160 through counter electrode 156. In another example of the disclosure, the sensor signal may be a voltage (e.g., Vcounter) measured at the working electrode 160 of glucose sensor electrodes 20 relative to counter electrode 156.

Measurement processor 132 receives the sensor signal (e.g., a measured current, voltage, and/or impedance) after the sensor signal is measured at glucose sensor electrodes 20 (e.g., a working electrode 160). Measurement processor 132 may receive the sensor signal and calibrate the sensor signal utilizing reference values. For example, measurement processor 132 may calibrate the sensor signal utilizing reference values based on a known analyte quantity, e.g., a zero glucose measurement to determine a baseline sensor signal. In some examples, changes to the sensor over time may change the responsivity of the glucose sensor, changing the sensor signal and glucose measurement accuracy. Measurement processor 132 may utilize the reference values to adjust for changes over time. In some examples, monitoring device 100 may update and or adjust the reference values, e.g., using electrochemical impedance spectroscopy (EIS) data or other data. In an example of the disclosure, the reference values are stored in a reference memory (e.g., memory 140) and provided to measurement processor 132. Based on the sensor signals and the reference values, measurement processor 132 may determine a glucose measurement. Measurement processor 132 store the glucose measurements in memory 140. The sensor measurements may be sent to display and transmission unit 134 to be either displayed on a display in a housing of monitoring device 100 or transmitted to an external device.

Memory 140 may be any type of memory device and may be configured to store glucose measurements produced by measurement processor 132, reference values used to determine glucose measurements from sensor signals, or other data used and/or produced by measurement processor 132 and/or controller 136. In some examples, memory 140 may further store software and/or firmware that is executable by measurement processor 132 and/or controller 136. For example, memory 140 may store interference detection unit 142. Interference detection unit 142 may be configured to be executable by measurement processor 132 and/or controller 136, and may include instructions for performing any of the techniques disclosed herein, for example, determining a glucose level based on signal generated by an electrochemical cell and a signal generated by a background electrode.

Sensor electronics device 130 may be a monitor which includes a display to display physiological characteristics readings. In some examples, sensor electronics device 130 may be remote from sensor set 10 and communicatively connected to sensor set 10, e.g., via a wired or wireless connection. For example, sensor electronics device 130 may also be installed in a desktop computer, a pager, a television including communications capabilities, a laptop computer, a server, a network computer, a personal digital assistant (PDA), a portable telephone including computer functions, an infusion pump including a display, a glucose sensor including a display, and/or a combination infusion pump/glucose sensor. Sensor electronics device 130 may be housed in a mobile phone, a network device, a home network device, or an appliance connected to a home network.

Power supply 138 may be a battery. The battery can include three series silver oxide 357 battery cells. In other examples, different battery chemistries may be utilized, such as lithium based chemistries, alkaline batteries, nickel metalhydride, or the like, and a different number of batteries may be used. Sensor electronics device 130 provides power to the sensor set 10 via power supply 138 through cable 102 and cable connector 104.

Controller 136 may be a processor, digital signal processors (DSPs), application specific integrated circuits (ASICs), field programmable gate arrays (FPGAs), or any other equivalent integrated or discrete logic circuitry. In some examples controller 136 may be configured to cause a specific voltage or current to be output to glucose sensor electrodes 20. Glucose sensor electrodes 20 may receive the voltage level or value. In an example of the disclosure, a reference electrode 158 of glucose sensor electrodes 20 may receive the reference voltage from power supply 138, which is referenced to ground. In addition, controller 136 may apply a voltage between working electrode(s) 160 and reference electrode 156, which allows current to be generated at the working electrode(s) 160 in the presence of glucose. That is, the application of the voltage level causes glucose sensor electrodes 20 to create a sensor signal (e.g., a current through a working electrode 160) indicative of a concentration of a physiological characteristic being measured (e.g., glucose). The current from the working electrode 160 used to measure the physiological characteristic (e.g., glucose) is referred to as iSig or Isig.

In accordance with the techniques, devices, and systems disclosed herein, monitoring device 100 may be configured to detect and compensate for electrochemical interference. In the examples disclosed herein, monitoring device includes a background electrode, and the background electrode is configured to be proximate electrochemical cell 18 and to detect an amount of electrochemical interference. In some examples, the background electrode may be substantially similar to glucose sensor electrodes 20 but without the glucose oxidase enzyme. For example, the background electrode may include an electrocatalytic layer catalyzing a reaction with the background electrode and an electrochemical interferant, such as acetaminophen, to generate an electrical signal corresponding to the electrochemical interferant, but will not catalyze a reaction with glucose. In other examples, the background electrode may have other differences from glucose sensor electrodes, e.g., omission of a glucose limiting membrane and/or adhesion layers, and/or differing electrocatalytic layer materials.

In some examples, the background electrode is configured to generate a signal that may be used to compensate for electrochemical interference included in iSig, e.g., a background signal that may be subtracted from iSig to remove a component of iSig caused by the electrochemical interference. For example, as described above, the iSig current is a sensor signal indicative of an amount of glucose (e.g., interstitial or possibly blood glucose). However, the iSig current may be actual iSig current plus current generated from an interferant (e.g., iInterferant). The term "actual iSig" is used to indicate the iSig that would be present if there were no interferants. Stated another way, the measured iSig may be actual iSig + iInterferant. In one or more examples, the signal generated by the background electrode may be indicative of iInterferant. Therefore, by subtracting iInterferant from measured iSig (e.g., iSig - iInterferant), processor 132 may determine the actual iSig, which represents the actual amount of glucose.

In some examples, a separate device including a background electrode configured to detect electrochemical interference may be included with glucose sensor 12. For example, a drug eluting device (not shown) may include both a steroid, such as dexamethasone, dexacetate, or the like, configured to compensate for a foreign body response of the patient and the background electrode. The drug eluting device may be configured to be mechanically compatible with glucose sensor 12 so as to be positioned at the insertion site along with glucose sensor 12. For example, the drug eluting device may comprise a flexible body, substrate, and/or thin film structure (e.g., polyimide) similar to glucose sensor 12 and configured to be positioned adjacent glucose sensor 12. In some examples, the drug eluting device may include the background electrode on a first side and/or surface (e.g., the "top" or "front" side) of the drug eluting device, and the drug and/or steroid may be a layer (e.g., coated) over at least a portion of the first side and/or surface and/or at least a portion of a surface of the background electrode. A second side (e.g., the "bottom" or "back" side) of the flexible body, substrate, and/or thin film structure of the drug eluting device opposite the first side may be configured to be adjacent to a second side (e.g., the "bottom" or "back" side) of glucose sensor 12. For example, the glucose sensor electrodes 20 may be disposed on a first side of a flexible body, substrate, and/or thin film structure (e.g., polyimide), and second side of the drug eluting device may be configured to be adjacent (e.g., contacting or noncontacting) the second side of glucose sensor 12, e.g., in a back-to-back configuration.

In other examples, glucose sensor 12 may include the background electrode. For example, the background electrode may be placed proximal to, between, or distal to one or more counter electrodes 156 and/or working electrodes 158, 160.

FIG. 2 is a block diagram illustrating example sensor electrodes and a bias voltage being applied to the sensor electrodes according to an example of the disclosure. In the examples shown, example sensor electrodes include counter electrode 156, reference electrode 158, and working electrodes 160, which may be examples of glucose sensor electrodes 20 and may be used in a glucose and oxygen sensor having a glucose oxidase (GOx) enzyme catalyzing a reaction with electrodes 156, 158, 160. In the example in FIG. 2, an operational amplifier (op amp) 150 or other servo-controlled device may connect to glucose sensor electrodes 20 through a circuit/electrode interface 152. Op amp 150, utilizing feedback through glucose sensor electrodes 20, attempts to maintain a prescribed voltage between reference electrode 158 and a working electrode 160 (e.g., VSET) by adjusting the voltage at counter electrode 156. In some examples, the voltage at reference electrode 158 is 850 mv. In some examples, different prescribed voltages (VSETs) may be applied to working electrodes 160, e.g., a first VSET applied to Working 1 and as second VSET applied to Working 2 in the example shown, relative to reference electrode 158.

Current 157 (iSig) may then flow from a counter electrode 156 to a working electrode 160. Counter electrode 156 balances the chemical reaction that is occurring at working electrode 160. Measurement processor 132 of FIG. 1 may measure current 157 to determine the electrochemical reaction between glucose sensor electrodes 20. As illustrate in FIG. 2, current 157 may be the iSig current. In some examples, the iSig current may be indicative of the amount of glucose that is present. However, in case of interferants, the iSig current (i.e., current 157) may be indicative of a combination of both the amount of glucose that is present and interferants. This disclosure describes examples for determining the amount of interferants, such as determining the amount that interferants are contributing to the iSig measurement, and subtracting that amount from the iSig measurement to determine the actual iSig measurement. The circuitry disclosed in FIG. 2 may be utilized in a long-term or implantable sensor or may be utilized in a short-term or subcutaneous sensor.

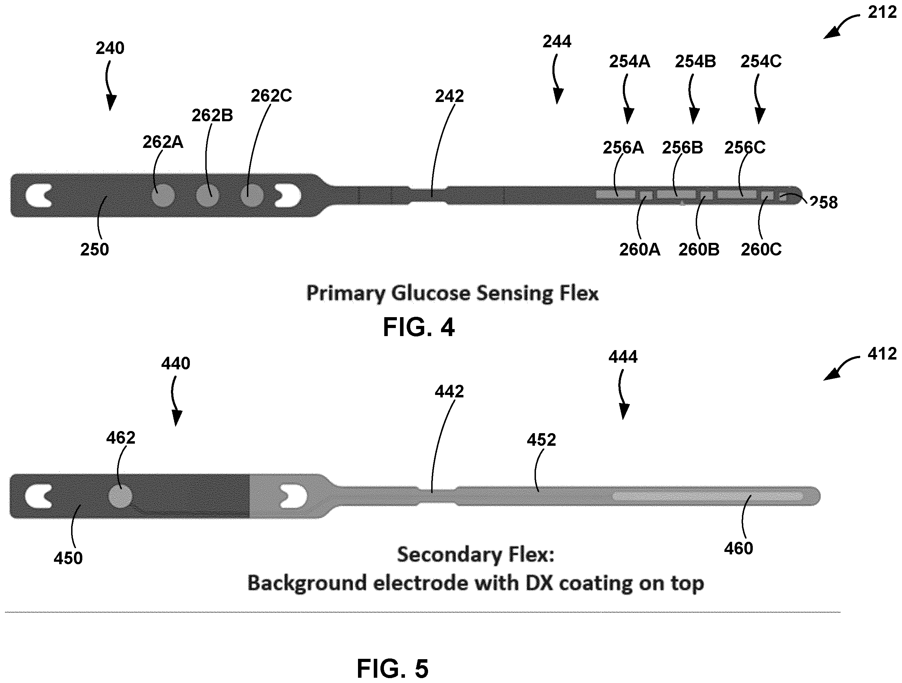

Returning to FIG. 1, as discussed above, due to electrochemical interference, monitoring device 100 may provide inaccurate readings to patient 112. As such, subcutaneous sensor set 10 may include one or more background electrode 120. Background electrode 120 is configured to be exposed to the bodily fluids, preferably proximate glucose sensor electrodes 20. Although illustrated as adjacent to an outer surface of cannula 16, background electrode 120 may alternatively be inside cannula 16 and configured to be exposed to bodily fluids through window 22, a separate window (not shown), or by any other suitable ways of exposing background electrode 120 to bodily fluids. In some examples, background electrode 120 may be disposed on a second body (an example of which is shown in FIG. 5 and other figures) separate from flexible thin film electrochemical glucose sensor 12, or separate from insertion needle 14. In the example shown, background electrode 120 is joined to connection portion 124 that terminates in conductive contact pads, or the like, and which may be substantially similar to connection portion 24 described above only connected to background electrode 120 rather than electrochemical cell 18 and possibly a separate conductive contact pad from the contact pads at which connection portion 24 terminates. In some examples, connection portion 124 may be a conductive trace, e.g., on a flexible insulative substrate.

In accordance with the techniques of this disclosure, background electrode 120 is configured to generate an electrical signal indicative of electrochemical interference proximate electrochemical cell 18. Background electrode 120 may be used in a variety of sensing applications and may be configured in a variety of ways. In some examples, background electrode 120 may be substantially similar to glucose sensor electrodes 20, e.g., working electrode 160, but without the biomolecule used as a catalytic agent. For example, background electrode 120 omit the glucose oxidase (GOx) enzyme of glucose sensor electrodes 20. Background electrode 120 may be placed in a human body in a vascular or non-vascular environment proximate to glucose sensor electrodes 20. For example, background electrode 120 may be placed in a vein and be subjected to a blood stream, or may be placed in a subcutaneous or peritoneal region of the human body, along with glucose sensor electrodes 20 and biomolecules.

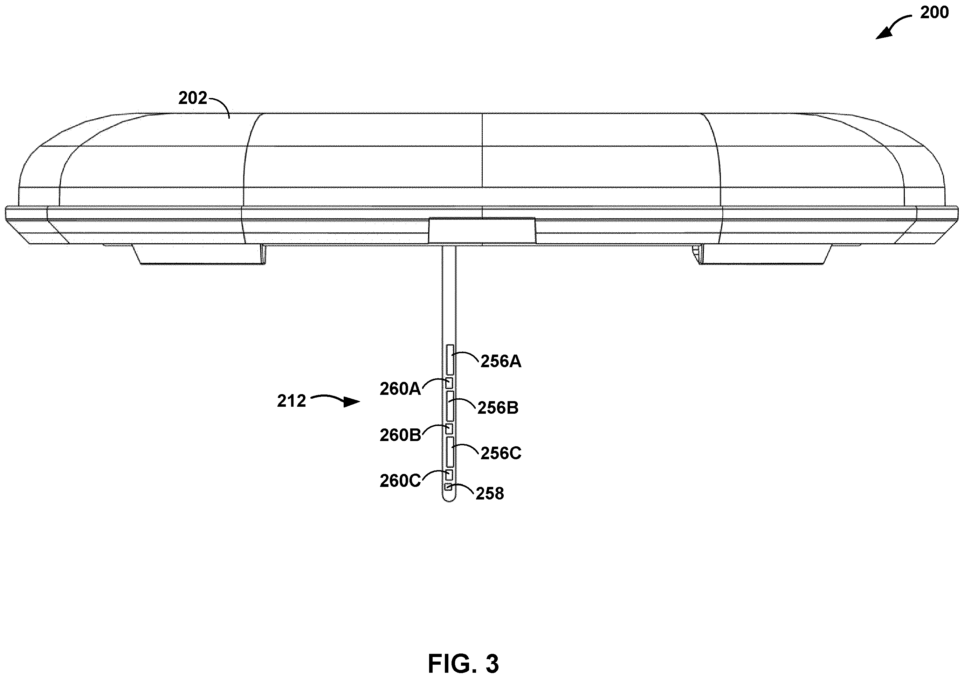

FIG. 3 is a cross-sectional view of an example medical device 200 including a glucose sensor 212. Glucose sensor 212 may be substantially similar to flexible thin film electrochemical glucose sensor 12 described above. In the example shown, medical device 200 includes housing 202 and glucose sensor 212.

Housing 202 may be configured to house a CGM (not shown) such that glucose sensor 212 may be inserted into a patient. Glucose sensor 212 may include sensor electrodes 256, 258, and 260. In the example shown, glucose sensor 212 includes three counter electrodes 256A, 256B, and 256C (collectively referred to as "counter electrodes 256"), and three working electrodes 260A, 260B, and 260C, (collectively referred to as "working electrodes 260"), which may form three electrochemical cells, e.g., working-counter electrode pairs providing three separate sensor signals (iSigs). Glucose sensor 212 also includes reference electrode 258. Each of counter electrodes 256, reference electrode 258, and working electrodes 260 may be substantially similar to counter electrode 156, reference electrode 158, and working electrodes 160 described above with reference to FIG. 2. Glucose sensor 212 may be connected to a CGM housed within housing 202. Although not shown in FIG. 3, but shown elsewhere, a background electrode like background electrode 120 may be position on glucose sensor 212, or on another substrate proximate glucose sensor 212.

FIG. 4 is a top view of an example glucose sensor 212. Glucose sensor 212 includes a proximal section 240, a bend portion 242, and a distal portion 244. The example glucose sensor 212 shown in FIG. 4 illustrates proximal portion 240, which is obscured in FIG. 3 by housing 202. The view of glucose sensor 212 illustrated in FIG. 4 may be a "top view" of a first side (alternatively referred to as a "top" side or "front" side) of glucose sensor 212 on which, and/or within which, electrochemical cells 254 are disposed. Glucose sensor 212 includes an opposing second side, alternatively referred to herein as the "bottom" side or "back" side of glucose sensor 212.

In the example shown, glucose sensor 212 comprises insulating body 250. In some examples, insulating body 250 may be an electrically insulating flexible body and/or substrate, such as a polyimide. In the example shown, insulating body 250 comprises the substrate for each of proximal portion 240, bend portion 242, and distal portion 244. Distal portion 244 includes electrodes 256, 258, and 260, and is configured to be inserted into a patient.

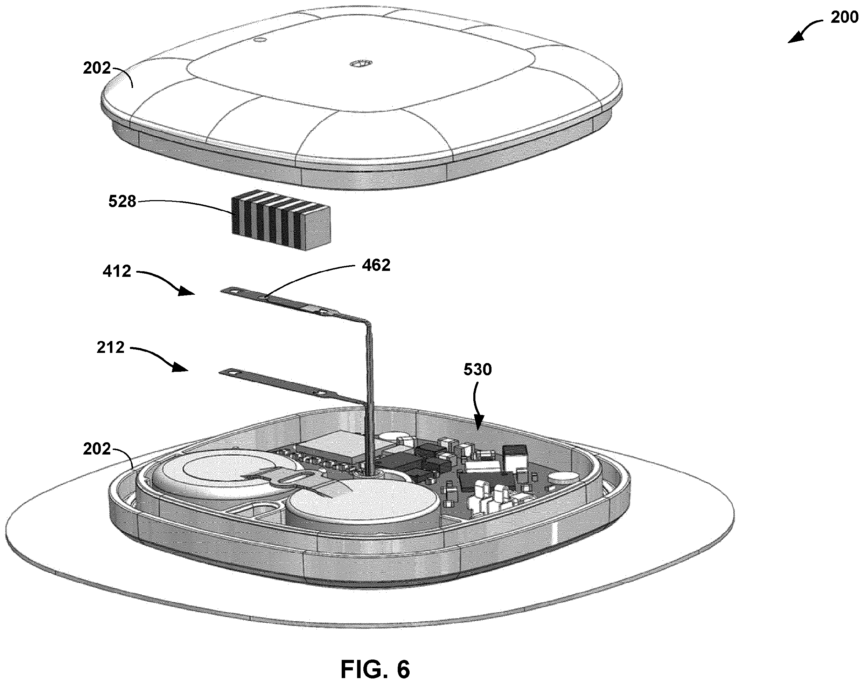

Proximal portion 240 includes contact pads 262A, 262B, and 262C (collectively "contact pads 262") respectively electrically connected to electrical traces (not shown) that are respectively electrically connected to the three electrochemical cells 254A, 254B, and 254C (collectively "electrochemical cells 254"), e.g., working-counter electrode pairs 260-256. Bend portion 242 is between proximal portion 240 and distal portion 244, and is configured to bend and/or allow proximal portion 240 to extend in a different direction than distal portion 244, as shown in FIG. 6 and described below. For example, bend portion 242 may be configured to allow proximal portion 240 and distal portion 244 to be at right angles, e.g., angled substantially at 90 degrees with respect to each other.

Contact pads 262 may be configured to electrically connect electrochemical cells 254 to an electrical circuit and/or sensor electronics, e.g., sensor electronics device 130, via a connector, e.g., connector block 28 described above and/or an elastomeric connector or a ZEBRA^{®} connector (FIG. 6). In some examples, an electrical circuit is configured to receive electrical signals indicative of the amount of glucose, e.g., the iSigs of electrochemical cells 254, via contact pads 262. In some examples, contact pads 262 may be dispose on a first side of insulating body 250 and may be the terminal ends of traces, or electrically connected to traces, that are electrically connected to electrochemical cells 254, e.g., carrying iSig from electrochemical cells 254 to contact pads 262.

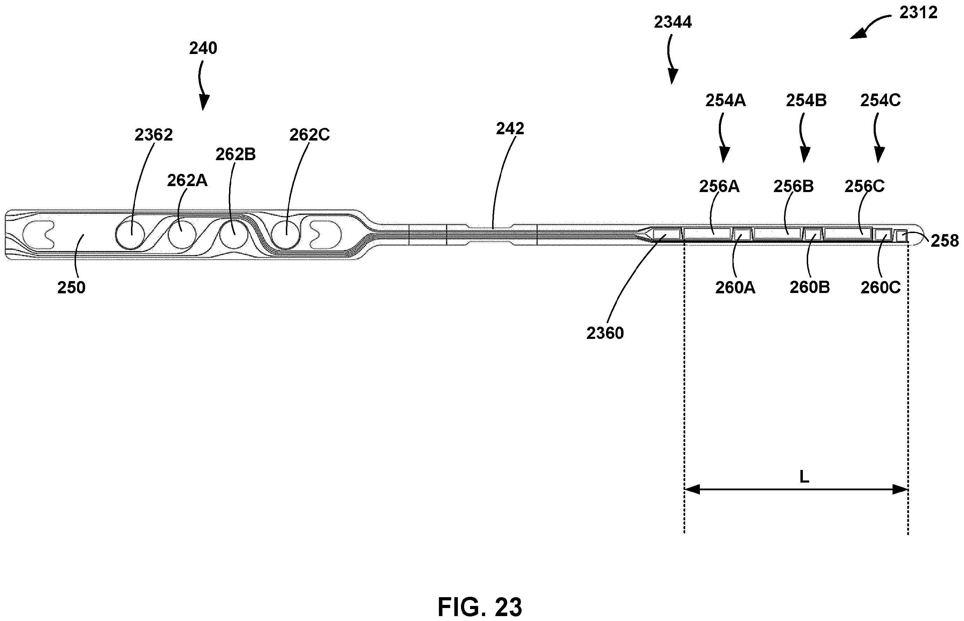

In some examples, glucose sensor 212 may include a background electrode configured to not catalyze a reaction with glucose and configured to generate a separate electrical signal (e.g., from iSig) indicative of an amount of electrochemical interference proximate any or all of electrochemical cells 254, such as described below with reference to FIGS. 23-27C. The background electrode may be electrically connected to a separate contact pad from contact pads 262, and a device such as monitoring device 100 and/or medical device 200 may determine an amount of glucose based on iSig generated by electrochemical cells 254 and the separate signal indicative of an amount of electrochemical interference from the background electrode, e.g., a portion of iSig may be generated by electrochemical interference rather than glucose and the signal generated by the background electrode may be used to correct iSig to an "actual" iSig.

In other examples, glucose sensor 212 is configured to be inserted into the patient along with another device, e.g., a drug eluting device including a background electrode configured to not catalyze a reaction with glucose and configured to generate the separate electrical signal indicative of an amount of electrochemical interference proximate any or all of electrochemical cells 254, such as drug eluting device 412 of FIG. 5 or any other drug eluting device described herein.

FIG. 5 is a top view of an example drug eluting device 412. Drug eluting device 412 includes a proximal section 440, a bend portion 442, and a distal portion 444. The view of drug eluting device 412 illustrated in FIG. 5 may be a "top view" of a first side (alternatively referred to as a "top" side or "front" side) of drug eluting device 412 on which, and/or within which, background electrode 460 is disposed. Drug eluting device 412 includes an opposing second side, alternatively referred to herein as the "bottom" side or "back" side of drug eluting device 412.

In the example shown, drug eluting device 412 comprises insulating body 450. In some examples, insulating body 450 may be an electrically insulating flexible body and/or substrate, such as a polyimide. In the example shown, insulating body 450 comprises the substrate for each of proximal portion 440, bend portion 442, and distal portion 444. Distal portion 444 includes background electrode 460 and is configured to be inserted into a patient.

Proximal portion 440 includes contact pad 462 electrically connected to an electrical trace 464 that is electrically connected to background electrode 460. Bend portion 442 is between proximal portion 440 and distal portion 444, and is configured to bend and/or allow proximal portion 440 to extend in a different direction than distal portion 444, as best seen below in FIG. 6. For example, bend portion 442 may be configured to allow proximal portion 440 and distal portion 444 to be at right angles, e.g., angled substantially at 90 degrees with respect to each other.

Contact pad 462 may be configured to electrically connect background electrode 460 to an electrical circuit and/or sensor electronics, e.g., sensor electronics device 130, via a connector, e.g., connector block 28 described above and/or an elastomeric connector or a ZEBRA^{®} connector (FIG. 6). In some examples, an electrical circuit is configured to receive electrical signals indicative of the amount of electrochemical interference, e.g., due to acetaminophen, via contact pad 462. In some examples, contact pad 462 may be disposed on the first side of insulating body 450 and may be a terminal end of electrical trace 464, or electrically connected to electrical trace 464, and electrical trace 464 may be electrically connected to background electrode 460, e.g., carrying an electrical signal (e.g., such as a current) indicative of an amount of electrochemical interference from background electrode 460 to contact pad 462.

Background electrode 460 is configured to not catalyze a reaction with glucose and is configured to generate an electrical signal indicative of an amount of electrochemical interference proximate an electrochemical cell, such as electrochemical cells 254 of glucose sensor 212. For example, background electrode 460 may not include a catalyst layer 812, e.g., a GOx layer, and drug eluting device 412 may be configured to be inserted into a patient along with, and proximate to, glucose sensor 212. The electrical signal that background electrode 460 is configured to generate may be indicative of an amount of electrochemical interference of any or all of electrochemical cells 254.

In some examples, at least a portion of drug eluting device 412 includes drug eluting layer 452. In the example shown, drug eluting layer 452 is disposed on or over the top side (e.g., the first side) of insulating body 450 and on or over an outer and/or top surface of background electrode 460. In some examples, drug eluting layer 452 may be disposed on or over the top side of just insulating body 450 or just background electrode 460. Drug eluting layer 452 may comprise a steroid, such as dexamethasone, dexacetate, or the like, configured to compensate for a foreign body response of the patient, e.g., in response to distal portion 244 of glucose sensor 212 and distal portion 444 of drug eluting device 412 being introduced into the patient. For example, drug eluting layer 452 may be configured to elute a drug, such as a steroid such as dexamethasone and/or dexacetate. Drug eluting layer 452 is configured to be exposed to fluids, e.g., blood, interstitial fluid, or the like, of the patient when at least a portion (e.g., distal portion 444) of drug eluting device 412 including drug eluting layer 452 is inserted into the patient. The fluids may act as a solvent extracting and/or removing the drug from drug eluting layer 452. The fluids may then absorb the drug configured to compensate for a foreign body response of the patient.

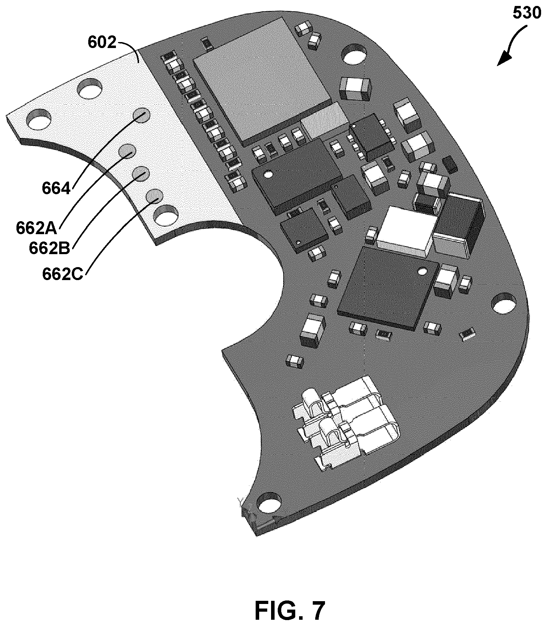

FIG. 6 is an exploded view of example medical device 200 including example glucose sensor 212 of FIG. 3 and drug eluting device 412 of FIG. 5. In the example shown, medical device 200 includes housing 202, glucose sensor 212, drug eluting device 412, connector 528, and electronical components 530. FIG. 7 is a perspective view of an example circuit board 602 of medical device 200, the example circuit board 602 including an electrical contact layout that may be used with the example glucose sensor 212 of FIG. 4 and drug eluting device 412 of FIG. 5. FIGS. 6 and 7 are described concurrently below.

Housing 202 may be configured to house electrical components 530, which may comprise CGM circuitry. Housing 202 may be configured to also house connector 528 and at least a portion of each of glucose sensor 212 and drug eluting device 412, e.g., at least proximal portions 240 and 440. Housing 202 may include a hole or aperture such that at least a portion of each of glucose sensor 212 and drug eluting device 412, e.g., distal portions 244 and 444, may extend through the hole or aperture and be inserted into a patient.

In the example shown, glucose sensor 212 and drug eluting device 412 are arranged to be adjacent to each other in a "back-to-back" configuration, e.g., in which the bottom and/or back sides (e.g., the opposing second sides) of insulating body 250 and insulating body 450 are facing each other and are adjacent to each other. In some examples, the back sides of each of insulating body 250 and insulating body 450 may be in contact with each other, e.g., when medical device 200 is assembled. In the back-to-back configuration shown, electrochemical cells 254 are disposed on the front side of insulating body 250 (e.g., as shown in FIG. 4) and background electrode 460 is disposed on the front side of insulating body 450 (e.g., as shown in FIG. 5), and are facing in opposite directions. That is, the front side of insulating body 250 faces a first direction, the first side of insulating body 450 faces a second direction opposite the first direction, and the back side of insulating body 250 is adjacent the back side of insulating body 450. In the example shown, contact pads 262 are not visible and are facing towards electrical components 530, and contact pad 462 is visible and is facing away from electrical components 530.

Connector 528 is configured to contact the top front side of insulating body 450 of drug eluting device 412 and an electrical contact configured to be connected to electrical components 530, e.g., via electrical contact 664 of circuit board 602 (FIG. 7) to which electrical components 530 are connected and/or mounted. In some examples, connector 528 may be an elastomeric connector or a ZEBRA^{®} connector. In some examples, circuit board 602 may be a printed circuit board (PCB).

In the example shown, because the front side of insulating body 250 is facing towards electrical components 503, e.g., towards circuit board 602 of electrical components 530, contact pads 262 may be configured to be in direct physical and electrical contact with electrical contacts 662 of circuit board 602. In contrast, in the back-to-back configuration shown, the front face of insulating body 450 is facing away from circuit board 602 and connector 528 is configured to electrically connect contact pad 462 to electrical contact 664 of the PCB.

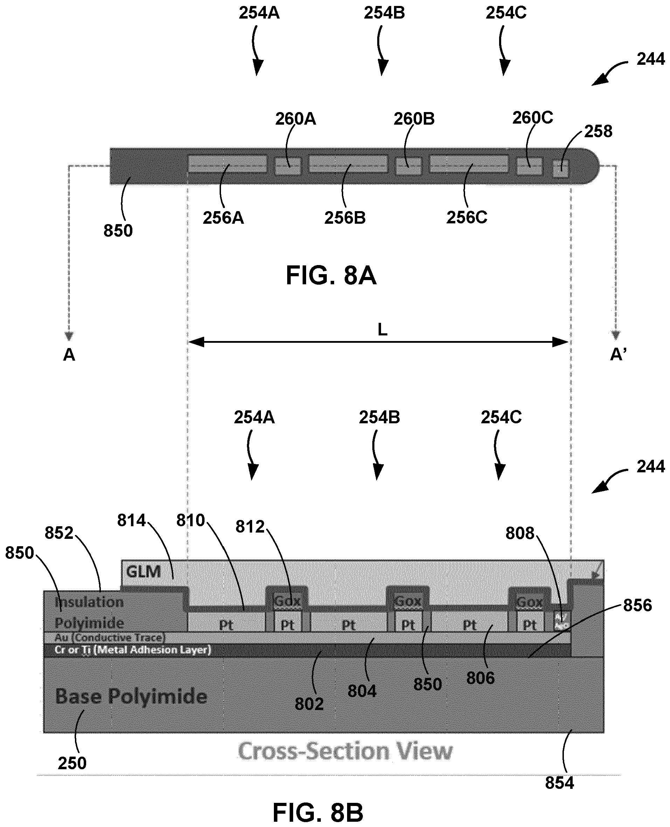

FIG. 8A is a top view of a distal portion 244 of example glucose sensor 212 of FIG. 4, and FIG. 8B is a cross-sectional view of the distal portion 244 of FIG. 8A taken along line A-A'. The examples shown in FIGS. 8A-8B illustrate example relative positioning and layer structure of glucose sensor 212.

In the examples shown, glucose sensor 212 includes insulating body 250. Insulating body 250 may be a flexible, electrical insulator such as a polyimide. Insulating body defines first side 852 (alternatively referred to as top side 852 or front side 852) and second side 854 (alternatively referred to as bottom side 854 or back side 854).

In the example shown, glucose sensor 212 includes metal adhesion layer 802 and conductive trace 804. Metal adhesion layer 802 is disposed on an intermediate surface 856 of insulating body 250 at a depth between first side 852 and second side 854. Conductive trace 804 is disposed on metal adhesion layer 802. Metal adhesion layer 802 is configured to promote and/or increase the adhesion of conductive trace 804 to the surface of insulating body 250. In some examples, metal adhesion layer 802 comprises chromium, and/or titanium, or any suitable material or compound configured to increase the adhesion of conductive trace 804 to insulating body 250.

Conductive trace 804 is configured to electrically connect electrochemical cells 254 and contact pads 262 at proximal portion 240 (not shown). In some examples, conductive trace 804 comprises one or more of gold, silver, aluminum, copper, platinum, or any suitable conductive material.

In some examples, glucose sensor 212 includes a plurality of metal adhesion layers 802 and conductive traces 804, e.g., each of which may be offset from each other in the width direction. For example, glucose sensor 212 may include individual conductive traces 804 for each electrochemical cell 254, e.g., three conductive traces 804 in the example shown. In some examples, glucose sensor 212 includes an additional conductive trace 804 configured to connect reference electrode 258 with one or more contact pads 262 or a separate contact pad (not shown), e.g., a fourth conductive trace to carry an electrical signal from reference electrode 258. In other examples, one or more of the electrical traces connected to electrochemical cells 254 is connected to reference electrode 258.

In some examples, intermediate surface 856 may be within a channel in insulating body 250. In other examples, intermediate surface 856 may be a surface of insulating body 250 that is subsequently covered via other layers. For example, insulating top layer 850 may be subsequently disposed on the intermediate surface layer of insulating body 250, e.g., via coating, lamination, deposition, or the like, to encapsulate metal adhesion layer 802 and conductive traces 804. Insulating top layer 850 may be substantially similar to insulating body 250 and may define a portion of top side 852 corresponding to the length and width of conductive traces 804. In some examples, insulating body 250 may be considered to include insulating top layer 850 in an arrangement configured to encapsulate conductive traces 804 within insulting body 250 at a depth between first side 852 and second side 854, e.g., to encapsulate and electrically isolate and/or insulate conductive traces 804 within polyimide.

In the examples shown, glucose sensor 212 includes electrocatalytic layer 806 disposed at least partially on a top surface of at least one conductive trace 804 and in electrical contact with the conductive trace 804. In some examples, electrocatalytic layer 806 may additionally disposed on an intermediate (e.g., depth-wise between first side 852 and second side 854) surface of insulating body 250, e.g., for an increased area along its length relative to conductive trace 804, e.g., the area corresponding to the areas of electrodes 260 and 256 illustrated in FIG. 8A. In some examples, the intermediate surface of insulating body 250 is at the same depth as the top surface of conductive traces 804, e.g., at widths adjacent to line A-A' and not visible in the cross-sectional view of FIG. 8B.

In some examples, electrocatalytic layer 806 comprises platinum. Electrocatalytic layer 806 is configured to generate an electrical signal, e.g., a current caused by a reaction with glucose catalyzed by glucose oxidase (GOx) layer 812. In the example shown, electrocatalytic layer 806 may be patterned to form electrochemical cells 254. For example, portions of insulating body 250 may be etched and/or otherwise patterned, and electrocatalytic layer 806 may be disposed (e.g., coated, vapor deposited, or the like) within the pattern to form the six electrocatalytic layers 806 areas corresponding to counter electrodes 260 and working electrodes 256 as illustrated in FIG. 8A, e.g., with insulating material, such as insulating body 250 and/or 850 separating the electrocatalytic layers 806 areas.

In the example shown, glucose sensor 212 includes catalyst layer 812 disposed on a top surface of at least a portion of electrocatalytic layer 806. In some examples, catalyst layer 812 may comprise a glucose oxidase (GOx) enzyme layer, and may be patterned. For example, catalyst layer 812 is disposed on the top surface of patterned electrocatalytic layer 806 corresponding to working electrodes 256. Catalyst layer 812 is configured to catalyze a reaction between glucose and electrocatalytic layer 806 causing an electrical signal (e.g., a current) at the electrocatalytic layer 806 proportional to the amount of glucose proximate electrochemical cells 254.

Glucose sensor 212 may include adhesion layer 810 configured to promote, improve, and/or increase adhesion of glucose limiting layer 814 to a top surface of catalyst layer 812 and/or electrocatalytic layer 816. Glucose limiting layer 814 is configured to reduce an amount of glucose that reaches catalyst layer 812, e.g., to an amount of glucose within the dynamic range of catalyst 812 and/or electrocatalytic layer 806 and/or to prolong the lifetime of catalyst layer 812. In some examples, glucose limiting layer 814 may be a glucose liming membrane. In some examples, glucose limiting layer 814 may be configured to encapsulate electrochemical cells 254, e.g., so as to limit the glucose that may come in contact with catalyst layer 812.

In the example shown, electrocatalytic layer 806, insulating body 250 and/or insulting top layer 850, and catalyst layer 812 may be patterned to form electrochemical cells 254 as illustrated in FIG. 8A. For example, electrochemical cell 254A may comprise counter electrode 260A and working electrode 256A which may be positioned proximal on insulating body 250 to electrochemical cell 254B. Electrochemical cell 254B may comprise counter electrode 260B and working electrode 256B which may be positioned proximal on insulating body 250 to electrochemical cell 254C. Electrochemical cell 254C may comprise counter electrode 260C and working electrode 256C which may be positioned proximal on insulating body 250 to reference electrode 258. Reference electrode 258 may be at or near a distal end of glucose sensor 212. Reference electrode 258 may comprise conductive layer 808, which may be disposed on at least portion of one of conductive traces 804 and/or at least a portion of an intermediate surface of insulating body 250, distal to electrochemical cells 254 as shown, and/or at substantially the same depth as electrocatalytic layer 806 (e.g., so as to contact a top surface of a conductive trace). In some examples, conductive layer 808 comprises silver, silver oxide, silver chloride and/or any other suitable conductive material.

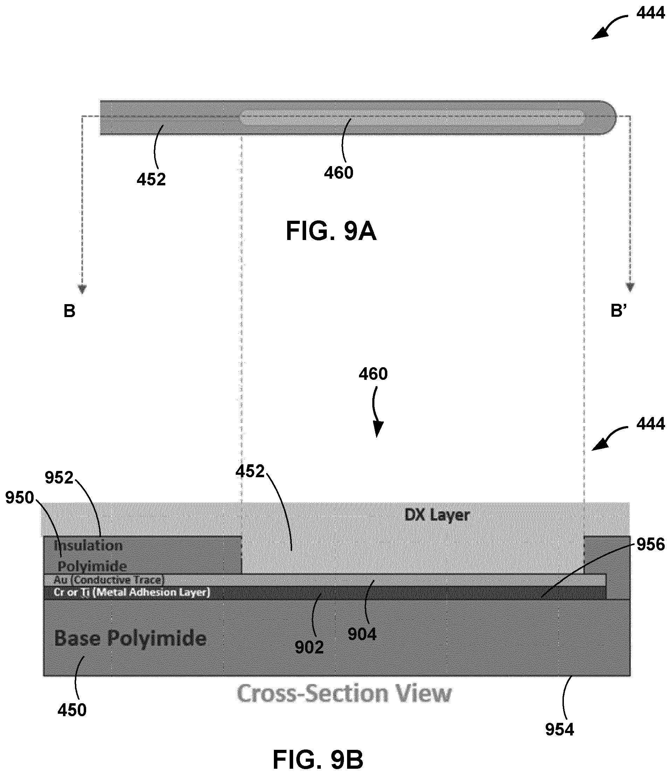

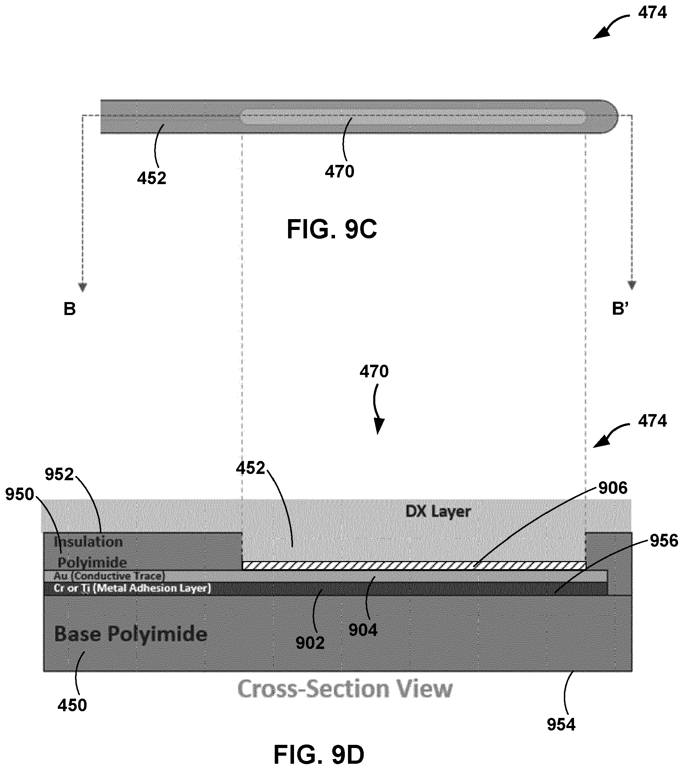

FIG. 9A is a top view of a distal portion 444 of example drug eluting device 412 of FIG. 5, and FIG. 9B is a cross-sectional view of the distal portion 444 of FIG. 9A taken along line B-B'. The examples shown in FIGS. 9A-9B illustrate example relative positioning and layer structure of drug eluting device 412.

In the examples shown, drug eluting device 412 includes insulating body 450. Insulating body 450 may be a flexible, electrical insulator such as a polyimide. Insulating body defines first side 952 (alternatively referred to as top side 952 or front side 952) and second side 954 (alternatively referred to as bottom side 954 or back side 954).

Drug eluting device 412 includes metal adhesion layer 902 and conductive trace 904, which may be substantially similar to metal adhesion layer 802 and conductive trace 804 of FIG. 8B. Metal adhesion layer 902 is disposed on an intermediate surface 956 of insulating body 450 at a depth between first side 952 and second side 954. Conductive trace 904 is disposed on metal adhesion layer 902. Metal adhesion layer 902 is configured to promote and/or increase the adhesion of conductive trace 904 to the surface of insulating body 450. In some examples, metal adhesion layer 902 comprises chromium, and/or titanium, or any suitable material or compound configured to increase the adhesion of conductive trace 904 to insulating body 450.

Conductive trace 904 is configured to electrically connect background electrode 460 and contact pad 462 at proximal portion 440 (not shown). In some examples, conductive trace 904 comprises one or more of gold, silver, aluminum, copper, platinum, or any suitable conductive material.

In some examples, intermediate surface 956 may be within a channel in insulating body 450. In other examples, intermediate surface 956 may be a surface of insulating body 450 that is subsequently covered via other layers. For example, insulating top layer 950 may be subsequently disposed on the intermediate surface layer of insulating body 450, e.g., via coating, lamination, deposition, or the like, to encapsulate metal adhesion layer 902 and conductive trace 904. Insulating top layer 950 may be substantially similar to insulating body 450 and may define a portion of top side 952 corresponding to the length and width of conductive trace 904. In some examples, insulating body 450 may be considered to include insulating top layer 950 in an arrangement configured to encapsulate conductive trace 904 within insulting body 450 at a depth between first side 952 and second side 954, e.g., to encapsulate and electrically isolate and/or insulate conductive trace 904 within polyimide.

Conductive trace 904 may have an area corresponding to the area of background electrode 460, e.g., as illustrated in FIG. 9A. In some examples, conductive trace 904 may be background electrode 460 for a portion of its length and/or area corresponding to background electrode 460, e.g., conductive trace 904 may be integral with background electrode 460, and conductive trace 904 may be encapsulated within insulating body 450 for a first portion of its length/area and may be exposed, e.g., not encapsulated within insulating body 450 for a second portion of its length/area defining and/or forming background electrode 460. Background electrode 460, e.g., the exposed area of conductive trace 904, may be configured to generate an electrical signal indicative of electrochemical interference, e.g., via an electrocatalytic reaction with an interferant such as acetaminophen.

In FIGS. 9A and 9B, drug eluting device 412 includes drug eluting layer 452. Drug eluting layer 452 may be configured to elute a drug, such as a steroid such as dexamethasone and/or dexacetate, as described above. For example, drug eluting layer 452 is configured to be exposed to fluids, e.g., blood, interstitial fluid, or the like, of the patient when at least a portion of drug eluting device 412 including drug eluting layer 452 is inserted into the patient. The fluids may act as a solvent extracting and/or removing the drug from drug eluting layer 452. In the example shown, drug eluting layer 452 is disposed on a top surface of background electrode 460, e.g., a top surface of the second portion of conductive trace 904. In the example shown, drug eluting layer 452 is disposed on at least a portion of the area of top side 952 of insulating body 450 and/or 850. In some examples, drug eluting layer 452 is disposed on top side 952 proximal to electrode 460, and in some examples drug eluting layer 452 is disposed on top side 952 distal to electrode 460, and in some examples, as in the example shown, drug eluting layer 452 is disposed on top side 952 proximal and distal to electrode 460.

In some examples, the surface area of background electrode 460 is greater than the surface area of one or more of electrochemical cells 254, alone or in combination. For example, if the length and width of distal portion 244 of glucose sensor 212 illustrated in FIG. 8A is substantially similar to the length and width of distal portion 444 of drug eluting device 212, background electrode 460 has a greater surface area than electrodes 256, 258, 260, alone or in combination, e.g., at least by virtue of being continuous and not segmented as electrodes 260/256 are in the examples shown.

FIG. 9C is a top view of a distal portion 474 of another example drug eluting device similar to drug eluting device 412 of FIG. 5, the difference being that distal portion 474 includes background electrode 470 rather than background electrode 460. FIG. 9B is a cross-sectional view of the distal portion 474 of FIG. 9C taken along line B-B'. The examples shown in FIGS. 9C-9D illustrate example relative positioning and layer structure of the drug eluting device similar to drug eluting device 412.

In the example shown, background electrode 470 is substantially similar to background electrode 460 except that background electrode 470 includes conductive layer 906. Conductive layer 906 may be the active electrode layer and/or surface of background electrode 470, and may be in electrical contact with conductive trace 904. Conductive layer 906 may comprise one or more of gold, silver, aluminum, copper, platinum, or any suitable conductive material.

In some examples, conductive layer 906 may have an area corresponding to the area of background electrode 470, e.g., as illustrated in FIG. 9C. In some examples, conductive layer 906 may be background electrode 470. Background electrode 470, e.g., the exposed area of conductive layer 906, may be configured to generate an electrical signal indicative of electrochemical interference, e.g., via an electrocatalytic reaction with an interferant such as acetaminophen, and conductive trace 904 may be configured to receive the electrical signal indicative of electrochemical interference, via electrical contact with conductive layer 906, and conduct the electrical signal to contact pad 462.

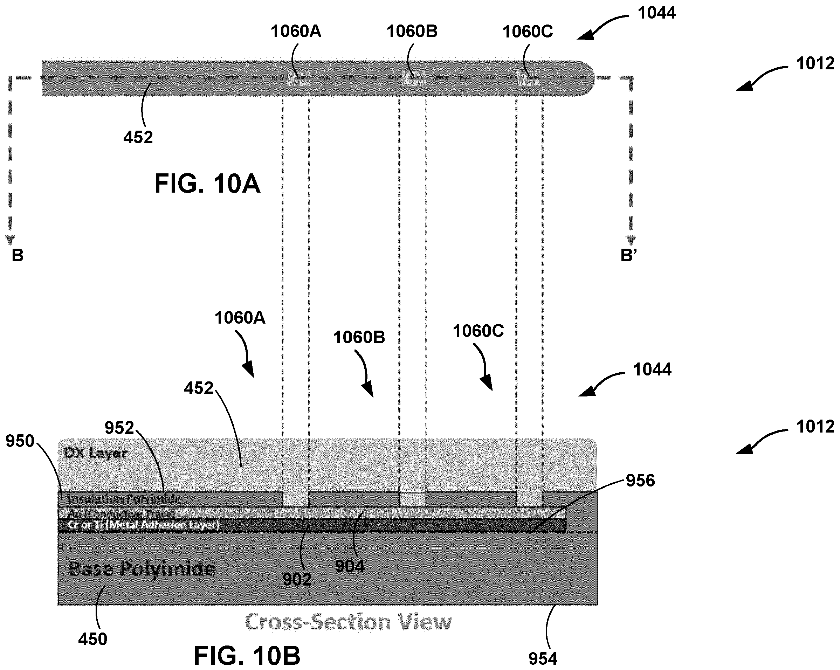

FIG. 10A is a top view of a distal portion 1044 of another example drug eluting device 1012, and FIG. 10B is a cross-sectional view of the distal portion 1044 of FIG. 10A taken along line B-B'. The examples shown in FIGS. 10A-10B illustrate example relative positioning and layer structure of drug eluting device 1012. Drug eluting device 1012 may be substantially similar to drug eluting device 412, except drug eluting device 1012 includes a plurality of background electrodes 1060A, 1060B, and 1060C (collectively "background electrodes 1060").

In the example shown, background electrodes 1060 may be substantially similar to background electrode 460 described above, e.g., defined and/or formed via an area of conductive trace 904 being exposed and not encapsulated within insulating body 450 and/or insulating top layer 950, except that background electrodes 1060 are a plurality of individual electrode areas. In the example shown, background electrode 1060A is positioned longitudinally along the length of drug eluting device 1012 proximal to background electrode 1060B, which is positioned proximal to background electrode 1060C, which is positioned relatively near and/or proximate to the distal end of drug eluting device 1012.

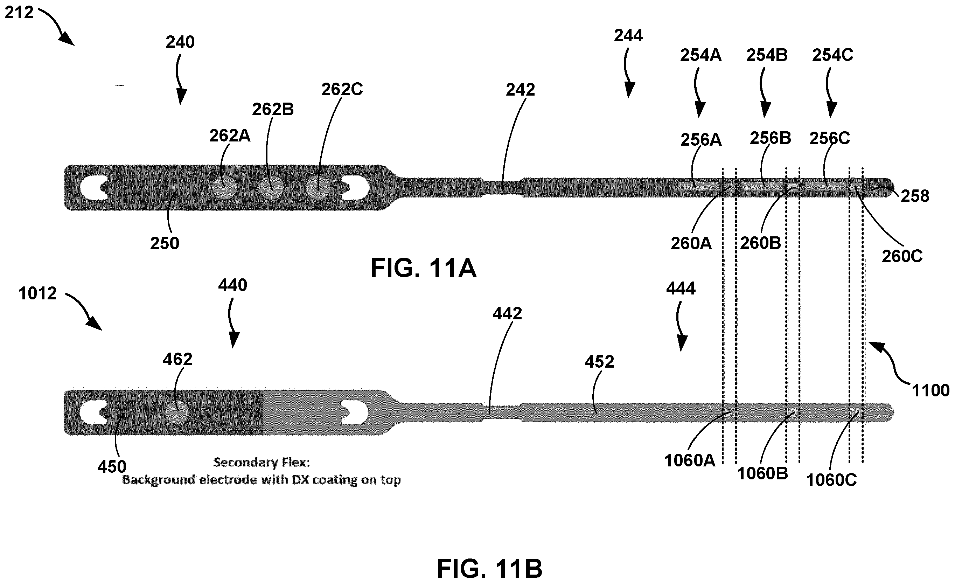

In the example shown, the areas and longitudinal positions of each of background electrodes 1060 correspond to the areas longitudinal positions of working electrodes 260 of glucose sensor 412, e.g., as shown in FIGS. 11A and 11B. FIG. 11A is a top view of example glucose sensor 212 of FIG. 8A, and FIG. 11B is a top view of the example drug eluting device 1012 of FIG. 10A and arranged for visual comparison with glucose sensor 212. In the examples shown, the relative sizes and lengths of glucose sensor 412 and drug eluting device 1012 are substantially the same, as are the positions and areas of working electrodes 260 and background electrodes 1060 as highlighted by lines 1100 between FIG. 11A and FIG. 11B. For example, background electrode 1060A is disposed at a first position along the length direction of insulating body 450, e.g., the length direction being along top side 952 and perpendicular the direction which top side 952, and background electrode 1060, is facing, as well as perpendicular to the opposite direction, e.g., the direction which bottom side 954 is facing. Working electrode 260A is disposed at the same first position along the length direction of insulating body 250, such that when assembled in the back-to-back configuration, background electrode 1060A is at the same length position as working electrode 260A and electrochemical cell 254A. As such, when inserted into the patient, background electrode 1060A and working electrode 260A/electrochemical cell 254A are positioned at the same depth within the patient. Additionally, background electrode 1060A has substantially the same area as working electrode 260A, e.g., so as to mimic and/or receive the same amount of electrochemical interferant, e.g., acetaminophen, as working electrode 260A and consequently generate an electrical signal due to electrochemical interference that is similar and/or the same as working electrode 260A and/or electrochemical cell 254A. Such an arrangement may have the benefit of easing and/or improving calibration of the electrochemical interference signal generated by background electrodes 1060 relative to the amount of iSig generated by electrochemical cells 254 due to electrochemical interference, e.g., by having background electrodes matching in area and depth position within the patient. Background electrodes 1060B and 1060C may be similarly matched with working electrodes 260B and 260C, respectively, and electrochemical cells 254B and 254C, respectively.

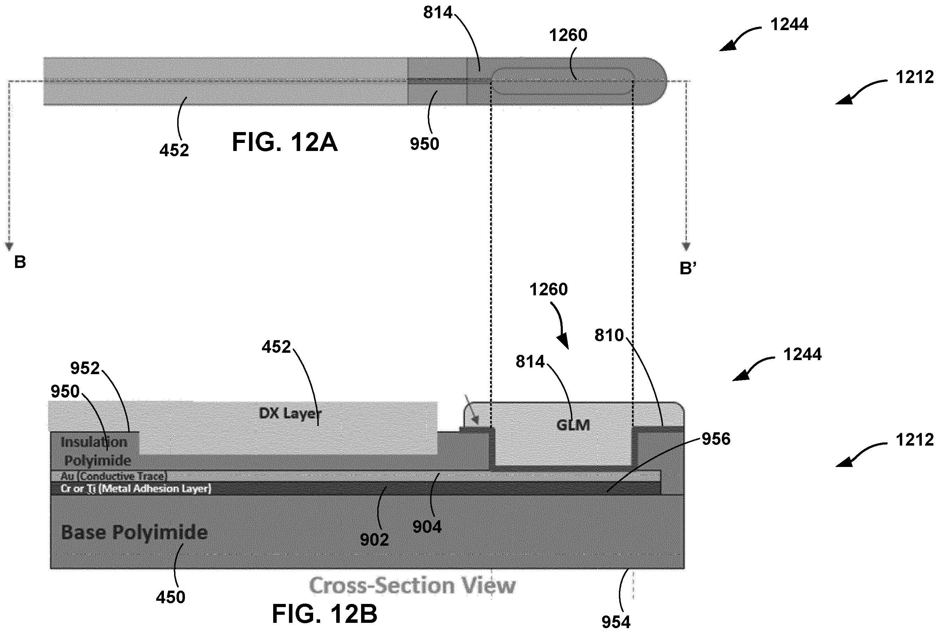

FIG. 12A is a top view of a distal portion 1244 of another example drug eluting device 1212, and FIG. 12B is a cross-sectional view of the distal portion 1244 of FIG. 12A taken along line B-B'. The examples shown in FIGS. 12A-12B illustrate example relative positioning and layer structure of drug eluting device 1212. Drug eluting device 1212 may be substantially similar to drug eluting device 412, except drug eluting device 1212 includes adhesion layer 810 disposed on background electrode 1260 (e.g., disposed on the top surface of conductive trace 904) and glucose limiting layer 814 disposed on adhesion layer 810.

In the example shown, background electrode 1260 may be substantially similar to background electrode 460 described above, e.g., defined and/or formed via an area of conductive trace 904 being exposed and not encapsulated within insulating body 450 and/or insulating top layer 950, except that background electrode 1260 further includes adhesion layer 810 and glucose limiting layer 814, e.g., as described above at FIGS. 8A and 8B. In other words, background electrode 1260 comprises glucose limiting layer 814 (e.g., a glucose limiting membrane) disposed over the top surface of background electrode 1260 (e.g., the top surface of conductive trace 904). Background electrode 1260 may provide the benefit of better mimicking the signal of electrochemical cells 254 caused by an electrochemical interferant, e.g., by including components of the layer structure of electrochemical cells 254, e.g., adhesion layer 810 and glucose limiting layer 814. Such components may change the amount and/or rate of electrochemical interferent reaching an electrocatalytic layer and/or conductive layer to cause an electrochemical interference signal.

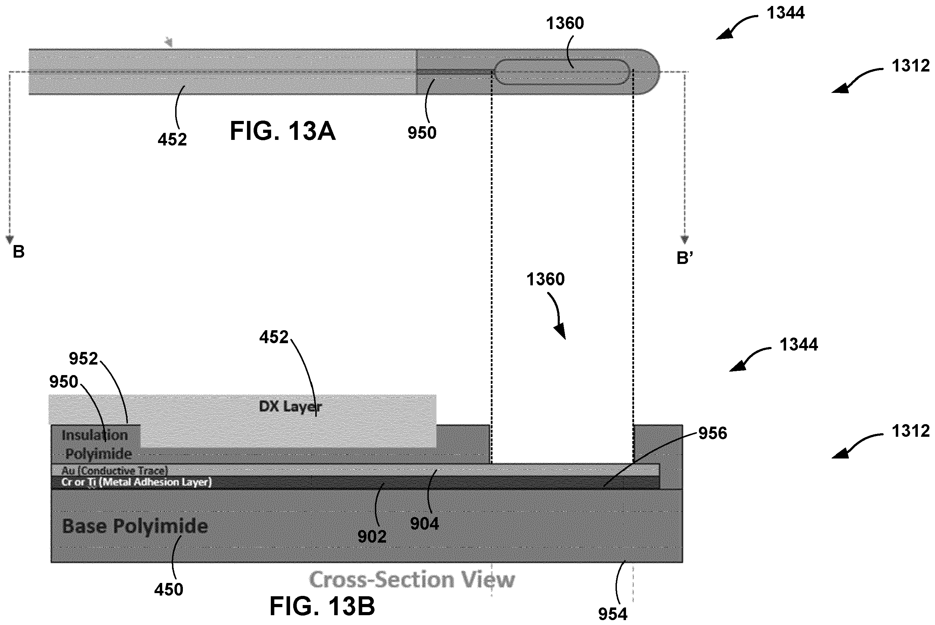

FIG. 13A is a top view of a distal portion 1344 of another example drug eluting device 1312, and FIG. 13B is a cross-sectional view of the distal portion 1344 of FIG. 13A taken along line B-B'. The examples shown in FIGS. 13A-13B illustrate example relative positioning and layer structure of drug eluting device 1312. Drug eluting device 1312 may be substantially similar to drug eluting device 1212, except drug eluting device 1312 does not include adhesion layer 810 or glucose limiting layer 814.

In the example shown, background electrode 1360 may be substantially similar to background electrode 460 described above, e.g., defined and/or formed via an area of conductive trace 904 being exposed and not encapsulated within insulating body 450 and/or insulating top layer 950. In the example shown, drug eluting layer 452 is disposed on and/or over first side 952 of insulating top layer 950 proximal to background electrode 1360. In some examples, top layer 950 may have a surface depth profile, e.g., a surface relief pattern in which the depth of the top surface of first side 952 varies over its area. In some examples, drug eluting layer 452 may be disposed on or over such a surface relief pattern and may have a top surface that is substantially the same as the surface relief pattern of first side 952, or drug eluting layer 452 may have a top surface that planarizes the surface relief pattern, e.g., as shown in FIG. 13B.

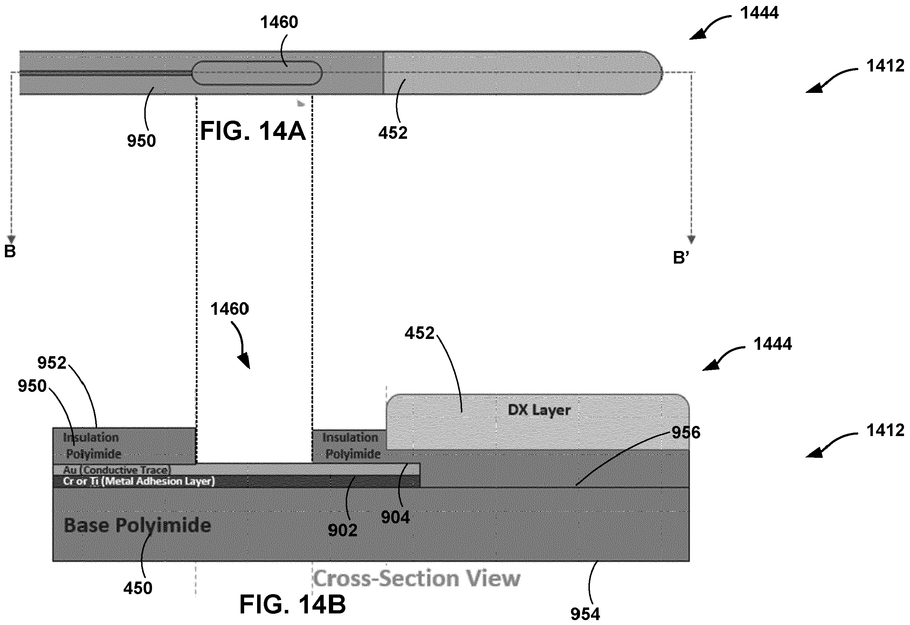

FIG. 14A is a top view of a distal portion 1444 of another example drug eluting device 1412, and FIG. 14B is a cross-sectional view of the distal portion 1444 of FIG. 14A taken along line B-B'. The examples shown in FIGS. 14A-14B illustrate example relative positioning and layer structure of drug eluting device 1412. In the example shown, background electrode 1460 may be substantially similar to background electrode 460 described above. Drug eluting device 1412 may be substantially similar to drug eluting device 1312, except that drug eluting layer 452 is disposed on and/or over first side 952 of insulating top layer 950 distal to background electrode 1460.

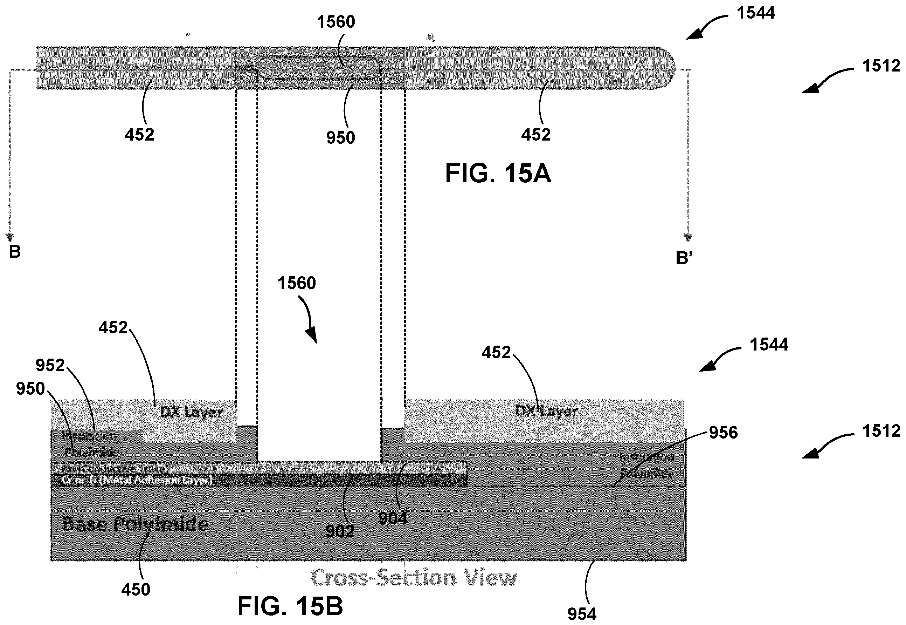

FIG. 15A is a top view of a distal portion 1544 of another example drug eluting device 1512, and FIG. 15B is a cross-sectional view of the distal portion 1544 of FIG. 15A taken along line B-B'. The examples shown in FIGS. 15A-15B illustrate example relative positioning and layer structure of drug eluting device 1512. In the example shown, background electrode 1560 may be substantially similar to background electrode 460 described above. Drug eluting device 1512 may be substantially similar to drug eluting devices 1212 and/ or 1312, except that drug eluting layer 452 is disposed on and/or over first side 952 of insulating top layer 950 distal to background electrode 1460.

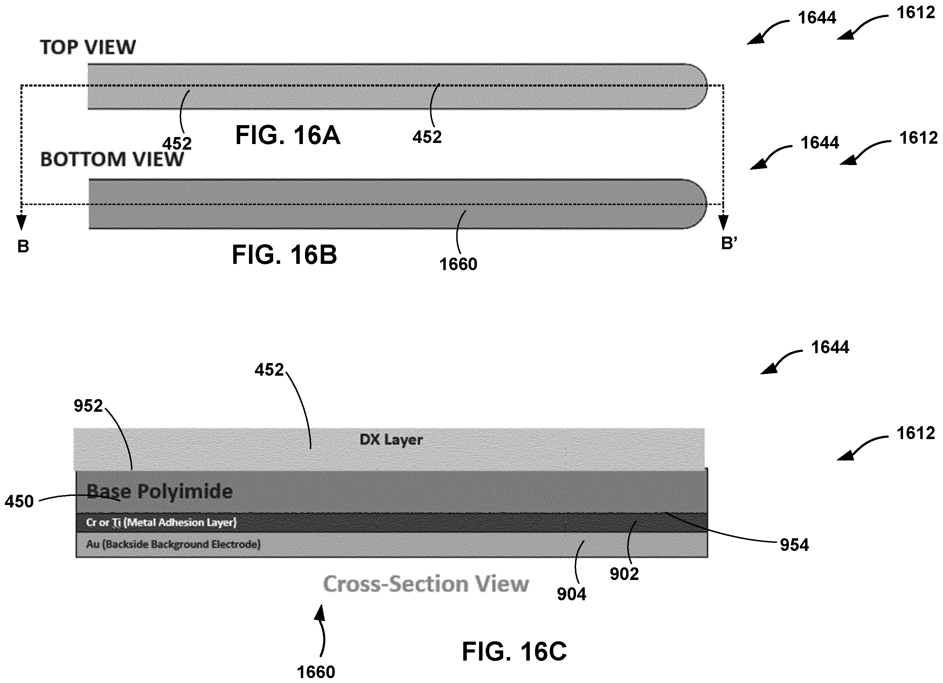

FIG. 16A is a top view of a distal portion 1644 of another example drug eluting device 1612, FIG. 16B is a bottom view of the distal portion 1644 of example drug eluting device 1612, and FIG. 16C is a cross-sectional view of the distal portion 1644 of FIGS. 16A and 16B taken along line B-B'. The examples shown in FIGS. 16A-16C illustrate example relative positioning and layer structure of drug eluting device 1612. Drug eluting device 1612 may be substantially similar to drug eluting device 412 of FIGS. 9A and 9B, except that background electrode 1660 is disposed on the second side 954 (e.g., bottom or back side 952) of insulating body 450 and not the first side 952 (e.g., top or front side 952).

In the example shown, metal adhesion layer 902 is disposed on second surface 954 of insulating body 450, and conductive trace 904 is disposed on metal adhesion layer 902. In some examples, background electrode 1660, e.g., at least a portion of conductive trace 904, may have an area similar to any of the example background electrodes disclosed herein, except background electrode 1660 is disposed on second side 954. In the example shown, the area of background electrode 1660 is substantially all of the area of second side 954 of distal portion 1644. In the example shown, drug eluting layer 452 is disposed on first side 952, which in the example shown in the first side (e.g., top and/or front side) of insulating body 450. In some examples, drug eluting layer 452 may have an area similar to any of the example drug eluting layers disclosed herein. In the example shown, the area of drug eluting layer 452 is substantially all of the area of first side 952 of distal portion 1644.

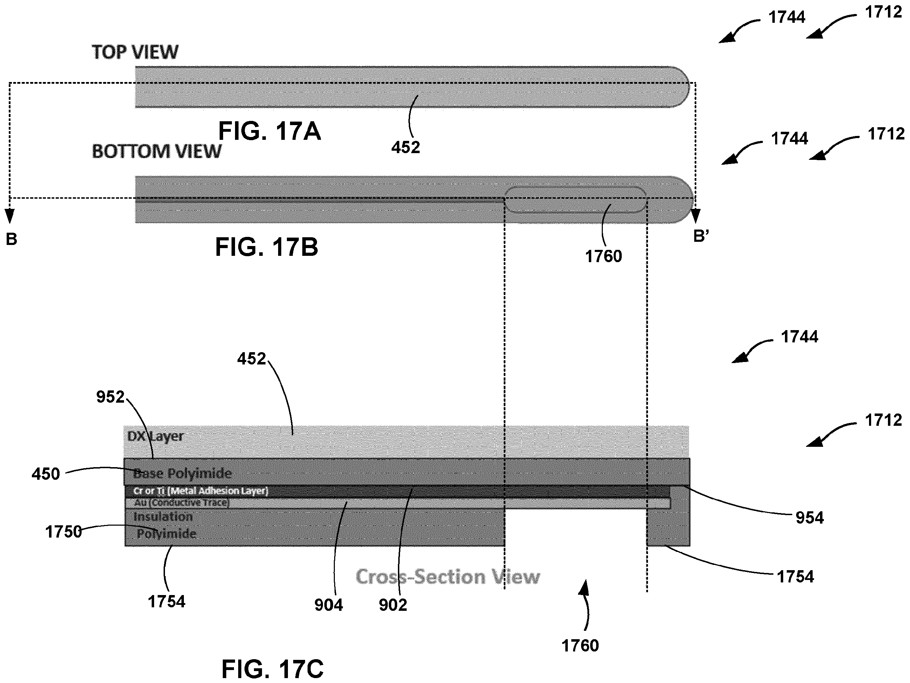

FIG. 17A is a top view of a distal portion 1744 of another example drug eluting device 1712, FIG. 17B is a bottom view of the distal portion 1744 of example drug eluting device 1712, and FIG. 17C is a cross-sectional view of the distal portion 1744 of FIGS. 17A and 17B taken along line B-B'. The examples shown in FIGS. 17A-17C illustrate example relative positioning and layer structure of drug eluting device 1712. Drug eluting device 1712 may be substantially similar to drug eluting device 1612, except that drug eluting device 1712 includes insulting bottom layer 1750 is disposed on and/or over at least a portion of conductive trace 904 on the second side 954. Insulating bottom layer 1750 may be substantially the same as insulating top layer 950, except that insulating bottom layer 1750 is disposed on and/or over at least a portion of a surface of conductive trace 904 and/or background electrode 1760 on the second side 954. In the example shown, second side 954 may be a surface of insulating body 450, and insulting bottom layer 1750 may define second side 1754 which may define an outer surface 1754 of drug eluting device 1712 facing the second direction, e.g., opposite the first direction in which first side 952 faces.

In some examples, background electrodes 1660 and 1760 may be "backside" electrodes, and background electrode 1760 may be a "segmented" backside electrode, e.g., having an area that is less than the second side 1754 area of distal portion 1744 and corresponding to an area of conductive trace 904 that is exposed and/or uncovered by insulating bottom layer 950.

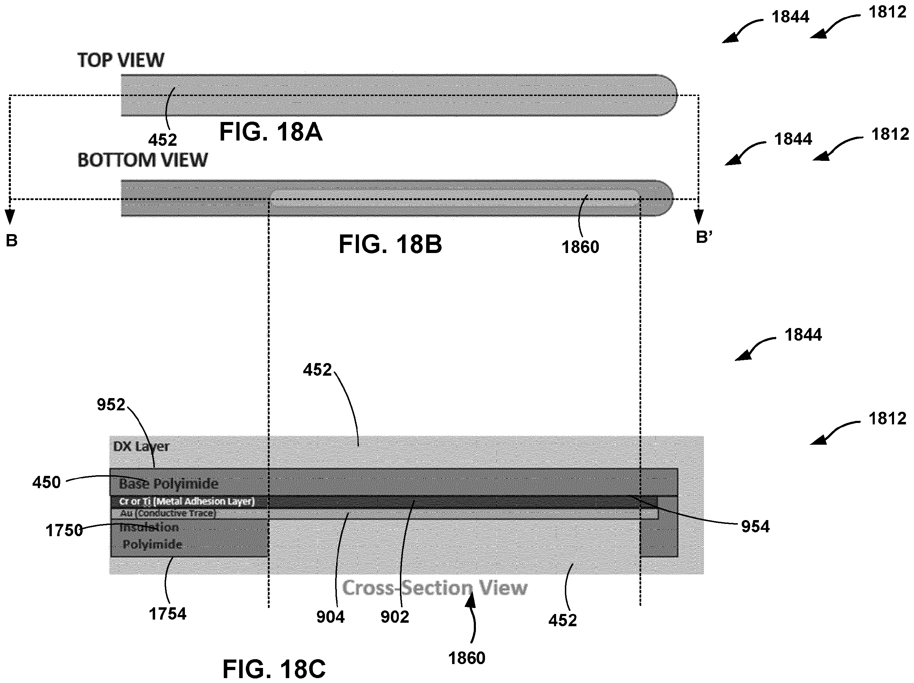

FIG. 18A is a top view of a distal portion 1844 of another example drug eluting device 1812, FIG. 18B is a bottom view of the distal portion 1844 of example drug eluting device 1812, and FIG. 18C is a cross-sectional view of the distal portion 1844 of FIGS. 18A and 18B taken along line B-B'. The examples shown in FIGS. 18A-18C illustrate example relative positioning and layer structure of drug eluting device 1812. In the example shown, drug eluting device 1812 includes background electrode 1860 which may be substantially similar to background electrode 1760 of FIGS. 17A-17C. Drug eluting device 1812 may be substantially similar to drug eluting device 1712, except that drug eluting layer 452 is disposed on second side 1754. In some examples, drug eluting layer 452 may be disposed on at least a portion of second side 1754, e.g., an outer surface of bottom insulting layer 1750, at least a portion of a surface of background electrode 1860, or both. In the example shown, drug eluting layer 542 is disposed on both first side 952 and second side 1754.

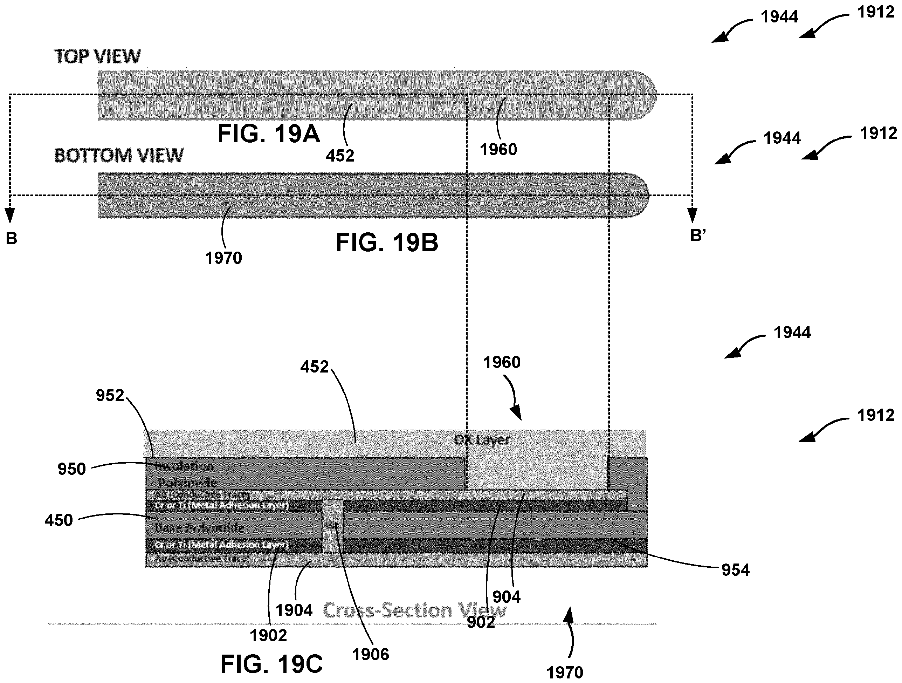

FIG. 19A is a top view of a distal portion 1944 of another example drug eluting device 1912, FIG. 19B is a bottom view of the distal portion 1944 of example drug eluting device 1912, and FIG. 19C is a cross-sectional view of the distal portion 1944 of FIGS. 19A and 19B taken along line B-B'. The examples shown in FIGS. 19A-19C illustrate example relative positioning and layer structure of drug eluting device 1912. In the example shown, drug eluting device 1912 includes background electrode 1960 which may be substantially similar to background electrode 1360 of FIGS. 13A-13C, and drug eluting device 1912 includes background electrode 1970 which may be substantially similar to background electrode 1660 of FIGS. 16A-16C.

In the example shown, drug eluting device 1912 may be substantially similar to a combination of drug eluting devices 1312 and 1612, e.g., including a first side 932 background electrode 1960 and a second side 954 background electrode 1970. In the example shown, background electrode 1970 comprises conductive trace 1904, which is disposed on metal adhesion layer 1902, which is disposed on second side 954 of insulating body 450. Conductive trace 1904 and metal adhesion layer 1902 may be substantially similar to conductive trace 904 and metal adhesion layer 902.

In some examples, background electrodes 1960 and 1970 may be combined to form a single background electrode, e.g., by way of being electrically connected by via 1906 as in the example shown. Via 1906 may comprise any suitable conductor, e.g., platinum, gold, silver, aluminum, copper, or the like. In other words, background electrode 1960 may be further disposed on second side 954 of insulating body 450. In other examples, background electrodes 1960 and 1970 may be separate background electrodes, e.g., drug eluting device 1912 may not include via 1906. For example, conductive traces 904 and 1904 may electrically connect background electrodes 1960 and 1970 to different contact pads (not shown) at a proximal portion of drug eluting device 1912, each of which may be substantially similar to contact pad 462 of FIG. 5. In other words, background electrode 1960 may be a first background electrode disposed on first side 952 of insulating body 450, and background electrode 1970 may be a second background electrode disposed on second side 954 of insulating body 450.

In the example shown, drug eluting device 1912 includes drug eluting layer 452. For example, drug eluting layer 452 is disposed at least partially on first side 952 and/or at least partially on a surface of background electrode 1960.

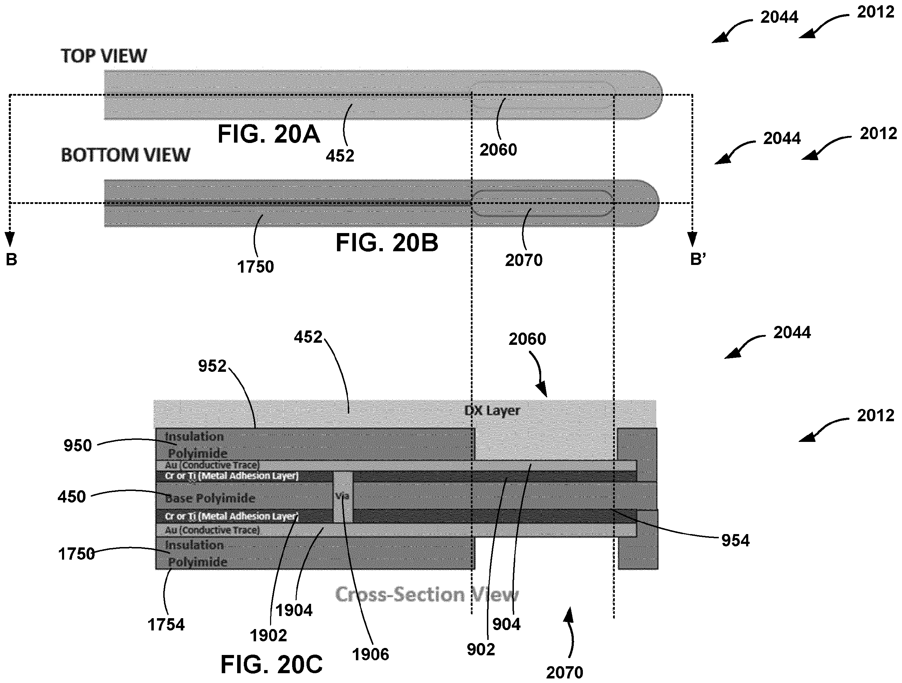

FIG. 20A is a top view of a distal portion 2044 of another example drug eluting device 2012, FIG. 20B is a bottom view of the distal portion 2044 of example drug eluting device 2012, and FIG. 20C is a cross-sectional view of the distal portion 2044 of FIGS. 20A and 20B taken along line B-B'. The examples shown in FIGS. 20A-20C illustrate example relative positioning and layer structure of drug eluting device 2012. In the example shown, drug eluting device 2012 includes background electrode 2060 which may be substantially similar to background electrode 1960 of FIGS. 19A-19C, and drug eluting device 2012 includes background electrode 2070 which may be substantially similar to background electrode 1970 of FIGS. 19A-19C. Drug eluting device 2012 may be substantially similar to drug eluting device 1912, except that drug eluting device 2012 includes insulting bottom layer 1750 disposed on and/or over at least a portion of conductive trace 1904 on the second side 954. Insulating bottom layer 1750 may be substantially the same as insulating top layer 950, except that insulating bottom layer 1750 is disposed on and/or over at least a portion of a surface of conductive trace 1904 and/or background electrode 2060 on the second side 954. In the example shown, second side 954 may be a surface of insulating body 450, and insulting bottom layer 1750 may define second side 1754 which may define an outer surface 1754 of drug eluting device 2012 facing the second direction, e.g., opposite the first direction in which first side 952 faces.

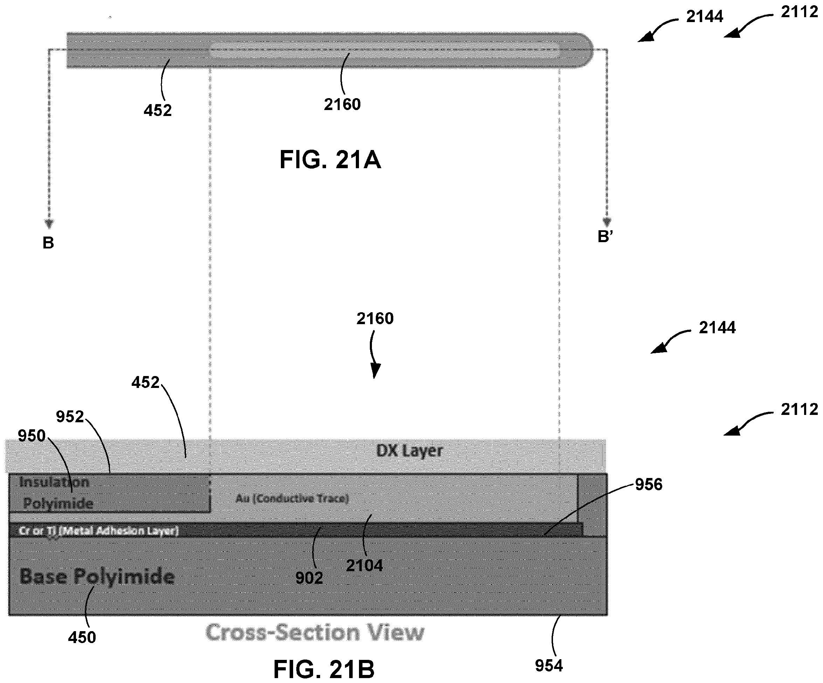

FIG. 21A is a top view of a distal portion 2144 of another example drug eluting device 2112, and FIG. 21B is a cross-sectional view of the distal portion 2144 of FIG. 21A taken along line B-B'. The examples shown in FIGS. 21A-21B illustrate example relative positioning and layer structure of drug eluting device 2112. Drug eluting device 2112 may be substantially similar to drug eluting device 912, except that background electrode 2160 has a thicker conductive area than background electrode 906. For example, drug eluting device 2112 includes conductive trace 2104 which may be substantially similar to conductive trace 904 except that the thickness and/or depth of conductive trace 2104 for a portion of its area corresponding to an active area and/or exposed area of background electrode 2160 at distal portion 2144 is increased, and in some examples an outer surface of conductive trace 2104 (e.g., and background electrode 2160) may be substantially coplanar with insulating top layer 950, e.g., coplanar with the outer surface of first side 954. In the example shown, insulating top layer 950 is disposed over a first portion of a surface of background electrode 2160, e.g., the thinner portion of conductive trace 2104 in the example shown.

In the example shown, drug eluting layer 452 is disposed over at least a portion of insulating top layer 950 and at least a portion of the area of background electrode 2160, e.g., at least a portion of the thicker area of conductive trace 2104. In some examples, drug eluting layer 452 is disposed over insulating top layer 950 at a first portion of distal portion 2144 and drug eluting layer 452 is disposed over a second portion of distal portion 2144 corresponding to at least a portion of the surface of background electrode 2160 and the thickness of drug eluting layer 452 is substantially the same at the first and second portions. In other words, in the example shown, drug eluting layer 452 has a substantially constant thickness and an outer surface at a substantially constant depth/thickness plane over at least portions of both insulating top layer 950 and the outer surface area of background electrode 2160.

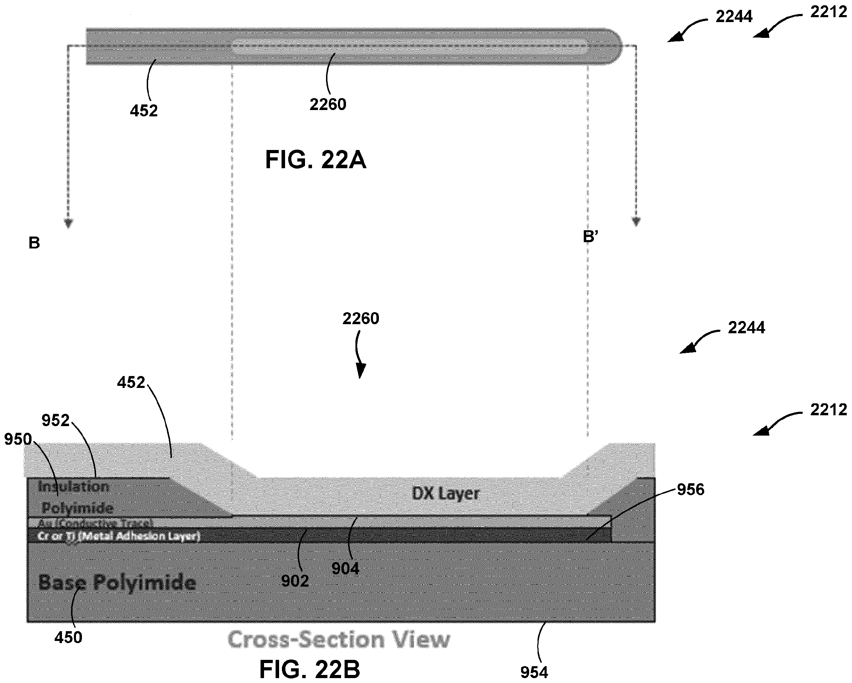

FIG. 22A is a top view of a distal portion 2244 of another example drug eluting device 2212, and FIG. 22B is a cross-sectional view of the distal portion 2244 of FIG. 22A taken along line B-B'. The examples shown in FIGS. 22A-22B illustrate example relative positioning and layer structure of drug eluting device 2212. Drug eluting device 2212 may be substantially similar to drug eluting device 912, except that insulating top layer 950 tapers in thickness between a first thickness and a zero thickness, e.g., at a perimeter of the active and/or exposed surface area of background electrode 2260 as in the example shown. In other words, background electrode 2260 is substantially similar to background electrode 2160 except that conductive trace 904 does not have a substantial increase in thickness at the active and/or exposed area of background electrode 2260, and insulating top layer 950 tapers down in thickness to a plane including the outer surface of conductive trace 904.

In the example shown, drug eluting layer 452 is disposed over first side 952 and over at least a portion of both top insulating layer 950 and at least a portion of the surface area of background electrode 2260 similar to drug eluting device 2112, except that drug eluting layer 452 follows the surface profile of the taper of insulating top layer 950. In some examples, n some examples, drug eluting layer 452 is disposed over insulating top layer 950 at a first portion of distal portion 2244 and drug eluting layer 452 is disposed over a second portion of distal portion 2244 corresponding to at least a portion of the surface of background electrode 2260 and the thickness of drug eluting layer 452 is substantially the same at the first and second portions. In other words, in the example shown, drug eluting layer 452 has a substantially constant thickness and an outer surface that changes depth/thickness plane when over a portion of insulating top layer 950 versus over the outer surface area of background electrode 2160, e.g., as illustrated in FIG. 22B.

FIG. 23 is a top view of another example glucose sensor 2312. Glucose sensor 2312 may be substantially similar to glucose sensor 212 of FIGS. 4, 8A, and 8B, except that glucose sensor 2312 includes background electrode 2360 and contact pad 2362.

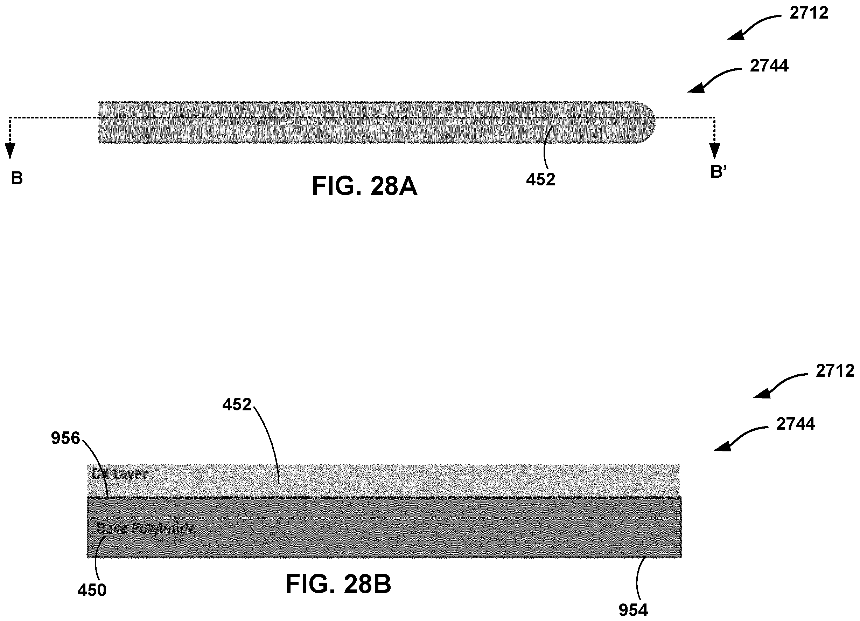

Background electrode 2360 may be substantially similar to any of background electrode 460, except with conductive trace 804 as the conductive surface of background electrode 460, or counter electrodes 260, reference electrodes 258, or working electrodes 256 except without adhesion layer 810 and/or GOx layer 812. In the example shown, background electrode 2360 is disposed on first side 852 of insulating body 850 and proximal to any or all of electrochemical cells 254. In some examples, the surface area, e.g., the active and/or exposed surface area of background electrode 2360 comprises an area that is from 20% to 50% a surface area of any one of electrochemical cell 254 or all of electrochemical cells 254 in combination. In the example shown, the longitudinal length of distal portion 2344 of glucose sensor 2312 is extended and/or lengthened, e.g., relative to distal portion 244 of glucose sensor 212. For example, distal portion 2344 is relatively longer than distal portion 244 via the inclusion of background electrode 2360. In the example shown, length L of distal portion 2360 corresponds to length L of distal portion 244 illustrated in FIGS. 8A and 8B. In some examples, glucose sensor 2312 may be used with drug eluting device 2812 of FIGS. 28A and 28B illustrated and described below, e.g., in medical device 200 and/or in a back-to-back configuration as described above.

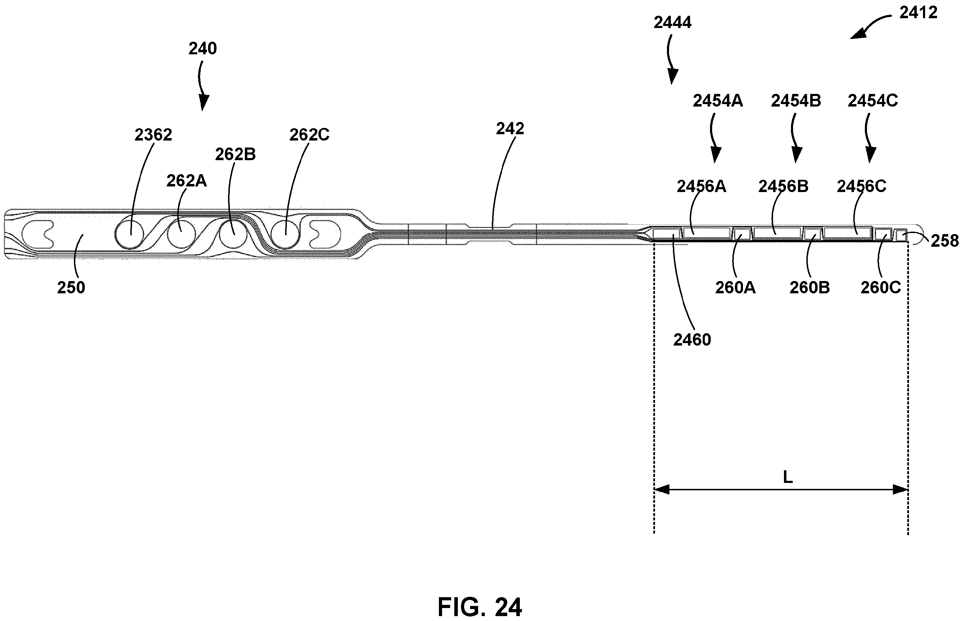

FIG. 24 is a top view of another example glucose sensor 2412. Glucose sensor 2412 may be substantially similar to glucose sensor 2312 of FIG. 23, except that background electrode 2460 is included in the longitudinal length L, e.g., that corresponds to the longitudinal length L of glucose sensor 212, and counter electrodes 2456 are reduced to accommodate for the area of background electrode 2460. In other words, glucose sensor 2412 may be substantially same length as glucose sensor 212 and include a background electrode 2460 substantially similar to background electrode 2360 of glucose sensor 2312, with counter electrodes 2456 being reduced relative to counter electrodes 256 to accommodate for the length of background electrode 2460. Electrochemical cells 2454 may be substantially similar to electrochemical cells 254, except having the reduced length counter electrodes 2461 rather than counter electrodes 260. In some examples, the surface area, e.g., the active and/or exposed surface area of background electrode 2460 comprises an area that is from 20% to 50% a surface area of any one of electrochemical cells 2454 or all of electrochemical cells 2454 in combination. In some examples, glucose sensor 2412 may be used with drug eluting device 2812 of FIGS. 28A and 28B illustrated and described below, e.g., in medical device 200 and/or in a back-to-back configuration as described above.

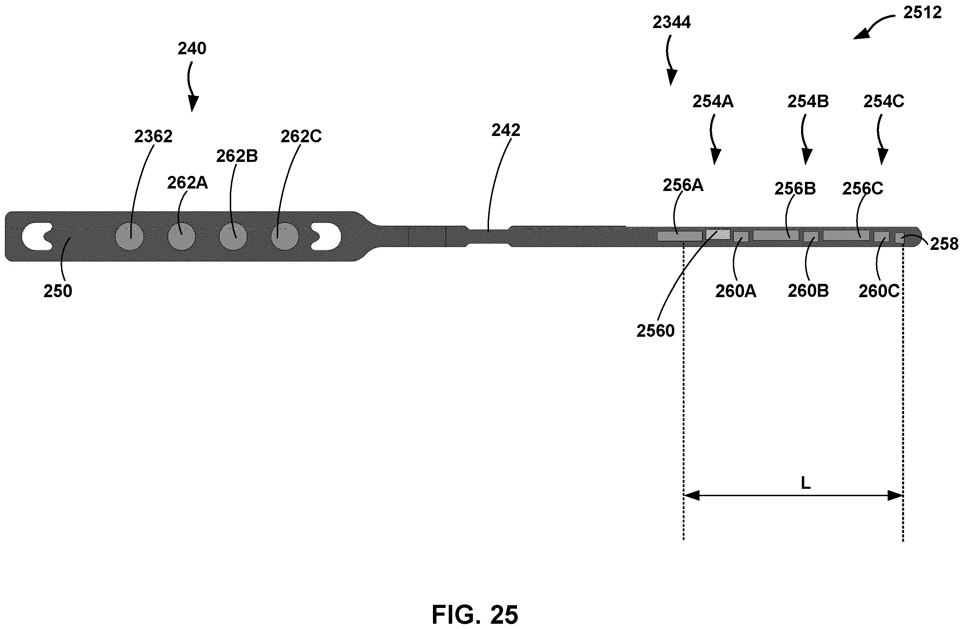

FIG. 25 is a top view of another example glucose sensor 2512. Glucose sensor 2512 may be substantially similar to glucose sensor 2312 of FIG. 23, except that glucose sensor 2512 includes background electrode 2560 positioned between counter electrode 256A and working electrode 260A rather than background electrode 2360. Background electrode 2560 may be substantially similar to background electrode 2360, except background electrode 2560 is positioned between counter electrode 256A and working electrode 260A.

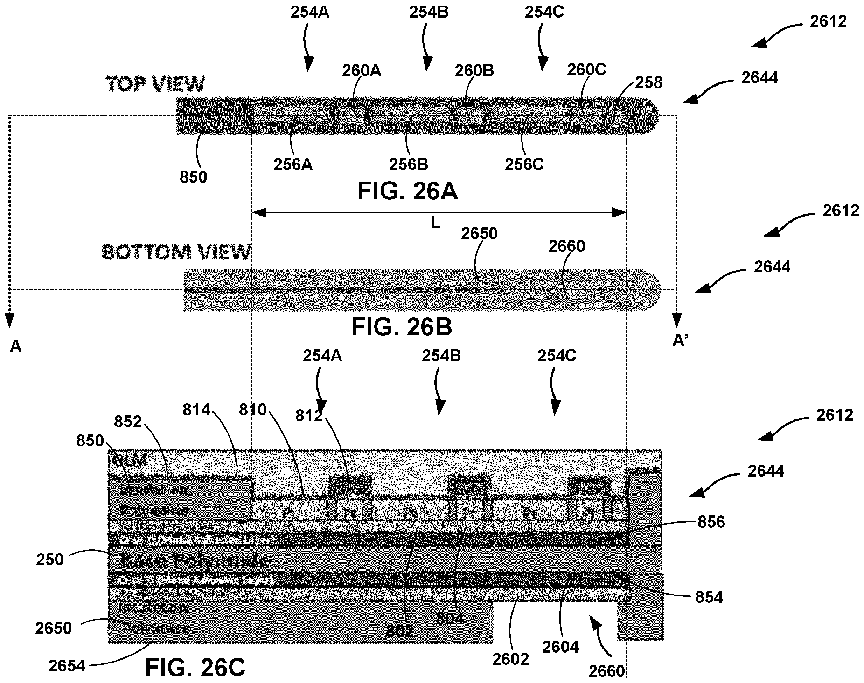

FIG. 26A is a top view of a distal portion 2644 of another example glucose sensor 2612, FIG. 26B is a bottom view of the distal portion 2644 of example glucose sensor 2612, and FIG. 26C is a cross-sectional view of the distal portion 2644 of FIGS. 26A and 26B taken along line A-A'. Glucose sensor 2612 may be substantially similar to glucose sensor 212 of FIGS. 4, 8A, and 8B, except that glucose sensor 2612 includes background electrode 2660 disposed on second side 854. In other words, glucose sensor 2612 includes electrochemical cells 254 disposed on a first side 852 of insulating body 250 and/or insulating top layer 850 and background electrode 2660 disposed on second side 854 of insulating body 250 opposite first side 852.

In the example shown, glucose sensor 2660 includes metal adhesion layer 2602 and conductive trace 2604 disposed on second side 854 of insulating body 250. In some examples, metal adhesion layer 2602 and conductive trace 2604 are substantially similar to metal adhesion layer 802 and conductive trace 804. In some examples, glucose sensor 2660 includes a fourth contact pad (not shown) electrically connected to background electrode 2660 by conductive trace 2604. Background electrode 2660 may be substantially similar to background electrode 2070 of FIG. 20C, only disposed on the second side 854 of insulating body 250 of glucose sensor 212 rather than a separate drug eluting device. In some examples, glucose sensor 2612 may be used with drug eluting device 2812 of FIGS. 28A and 28B illustrated and described below, e.g., in medical device 200 and/or in a back-to-back configuration as described above.

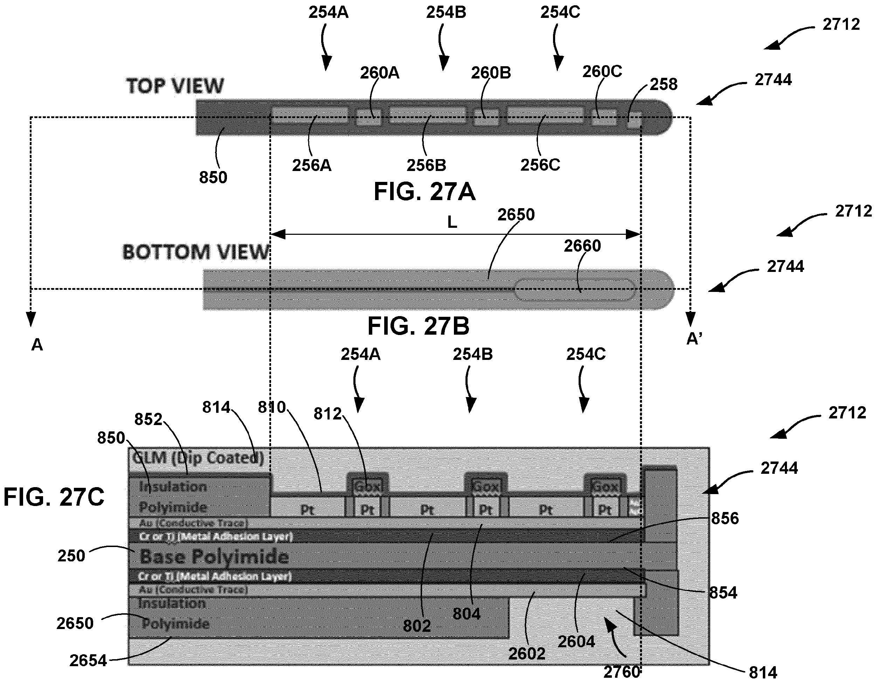

FIG. 27A is a top view of a distal portion 2744 of another example glucose sensor 2712, FIG. 27B is a bottom view of the distal portion 2744 of example glucose sensor 2712, and FIG. 27C is a cross-sectional view of the distal portion 2744 of FIGS. 27A and 27B taken along line A-A'. Glucose sensor 2712 may be substantially similar to glucose sensor 2612 of FIGS. 26A-26C, except that background electrode 2760 includes glucose limiting layer 814 disposed on and/or over at least a portion of the surface of background electrode 2760. In some examples, background electrode 2760 is substantially similar to background electrode 2660, except background electrode 2760 includes glucose limiting layer 814 dispose on at least a portion of its outer surface.

FIG. 28A is a top view of a distal portion 2844 of another example drug eluting device 2812, and FIG. 28B is a cross-sectional view of the distal portion 2844 of FIG. 28A taken along line B-B'. The examples shown in FIGS. 28A -28B illustrate example relative positioning and layer structure of drug eluting device 2812. Drug eluting device 2812may be substantially similar to drug eluting device 412, except drug eluting device 2812does not include a background electrode.

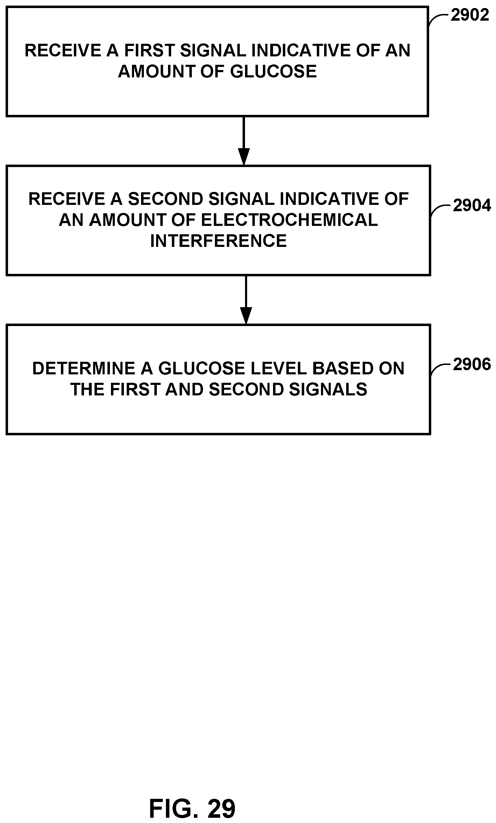

FIG. 29 is a flowchart illustrating an example method of determining a glucose level of a fluid of a person. Although the example technique of FIG. 29 is described with respect to system 110, monitoring devices 100 and/or 200, sensor electronics device 130 of FIGS. 1 and 2, glucose sensor 212 of FIGS. 8A and 8B, and drug eluting device 412 of FIGS. 9A and 9B, the example technique of FIG. 29 may be performed with any type of monitoring device and/or glucose sensor, e.g., including processing circuitry such as measurement processor 132 and/or controller 136, and/or any glucose sensor and/or drug eluting device disclosed here. In some examples, the example technique of FIG. 29 may be performed by processing circuitry (e.g., measurement processor 132), configured to execute instructions stored in memory (e.g., memory 140), such as interference detection unit 142.

Measurement processor 132 may receive a first signal indicative of an amount of glucose in a fluid of a person from an electrochemical cell (2902). For example, measurement processor 132 receive iSig from one or more of electrochemical cells 254. Measurement processor 132 may receive a second signal indicative of an amount of electrochemical interference in a fluid of a person from a background electrode (2904). For example, measurement processor 132 receive a current signal other than iSig from background electrode 460. In some examples, background electrode 460 is configured to not catalyze a reaction with glucose, and is positioned proximate and/or relatively close to one or more of electrochemical cells 254. In some examples, electrochemical cells 254 may be disposed on a first side of glucose sensor 212 that is separate from drug eluting device 412, and background electrode 460 is disposed on a first side of drug eluting device 412. In some examples, glucose sensor 212 and drug eluting device 412 are arranged in a back-to-back configuration, e.g., as described above.

Measurement processor 132 may determine a glucose level of the fluid of the person based on the first and second signals (2906). For example, measurement process 132 may subtract the second signal from the first signal to determine a glucose level of the fluid of the person. In some examples, measurement processor 132 may adjust and/or "weight" each of the first and second signals with the same or different adjustments and/or weights, e.g., via a calibration configured to adjust for differences in electrochemical cells 254 generating a signal due to electrochemical interference (e.g., in addition to the signal generated due to glucose) and background electrode 440 generating the second signal due to electrochemical interference.

Other illustrative examples of the disclosure are described below.

Example 1: A device includes: an electrochemical cell configured to generate a first electrical signal indicative of an amount of glucose in a fluid of a person, the electrochemical cell comprising a working electrode, a counter electrode, and a reference electrode; and a background electrode configured to not catalyze a reaction with glucose and is configured to generate a second electrical signal indicative of an amount of electrochemical interference proximate the electrochemical cell.

Example 2: The device of example 1, wherein the electrochemical cell is disposed on a first side of a first body, wherein the background electrode is disposed on a first side of a second body separate from the first body.

Example 3: The device of example 2, wherein the first side of the first body faces a first direction, wherein the first side of the second body faces a second direction opposite the first direction, wherein a second side of the first body opposite the first side of the first body is adjacent a second side of the second body, the second side of the second body opposite the first side of the second body.

Example 4: The device of example 3, wherein a drug eluting layer is disposed on the first side of the second body.

Example 5: The device of example 4, wherein the drug eluting layer is disposed on the first side of the second body proximal to the background electrode.

Example 6: The device of example 4, wherein the drug eluting layer is disposed on the first side of the second body distal to the background electrode.

Example 7: The device of example 4, wherein the drug eluting layer is disposed on the first side of the second body proximal and distal to the background electrode.

Example 8: The device of any one of examples 3 through 7, wherein a surface area of the background electrode is greater than a surface area of the electrochemical cell.

Example 9: The device of any one of examples 3 through 8, wherein the background electrode is disposed at a first position along a length direction of the second body, the length direction perpendicular to the first and second directions, wherein the electrochemical cell is disposed at the first position along the first body.

Example 10: The device of any one of examples 4 through 9, wherein the drug eluting layer is disposed on at least a portion of the surface of the background electrode.

Example 11: The device of any one of examples 3 through 10, wherein the background electrode comprises a glucose limiting membrane disposed over the background electrode surface.

Example 12: The device of any one of examples 3 through 11, wherein the background electrode is disposed on the second side of the second body and not the first side of the second body.

Example 13: The device of any of examples 11 and 12 or any of examples 11 and 12, wherein an insulator is disposed over at least a portion of a surface of the background electrode.

Example 14: The device of any one of examples 4 through 13, wherein the background electrode is further disposed on a second side of the second body.

Example 15: The device of example 14, wherein an insulator is disposed over a portion of a surface of the background electrode on the second side of the second body.

Example 16: The device of any one of examples 4 through 15, wherein an insulator is disposed over a first portion of a surface of the background electrode, wherein a second portion of the surface of the background electrode is coplanar with the insulator.

Example 17: The device of any one of examples 4 through 16, wherein an insulator is disposed over a first portion of a surface of the background electrode, wherein the drug eluting layer is disposed over the insulator at the first portion and over a second portion of the surface of the background electrode, wherein the thickness of the drug eluting layer is the same at the first and second portions.

Example 18: The device of any one of examples 1 through 17, wherein the background electrode is proximal the electrochemical cell.

Example 19: The device of example 18, wherein a surface area of the background electrode comprises an area that is from 20% to 50% a surface area of the electrochemical cell.

Example 20: The device of any one of examples 1 through 19, wherein the background electrode is positioned between the counter electrode and the working electrode.

Example 21: The device of any one of examples 1 through 20, wherein the electrochemical cell is disposed on a first side of a first body, wherein the background electrode is disposed on a second side of the first body opposite the first side.

Example 22: The device of example 21, wherein the electrochemical cell comprises a glucose limiting membrane, wherein the background electrode comprises the glucose limiting membrane.

Example 23: The device of any one of examples 1 through 22, wherein the electrochemical interference is caused by acetaminophen.

Example 24: The device of any one of examples 1 through 23, wherein the surface of the electrode comprises an electrocatalytic material.

Example 25: The device of example 24, wherein the electrocatalytic material is at least one of gold or platinum.

Example 26: The device of any one of examples 4 through 25, wherein the drug eluting layer comprises at least one of dexamethasone or dexacetate.

Example 27: A glucose monitor system includes: an electrochemical cell configured to generate a first electrical signal indicative of an amount of glucose in a fluid of a person, the electrochemical cell disposed on a first side of a first insulative substrate; a background electrode configured to not catalyze a reaction with glucose and is configured to generate a second electrical signal indicative of an amount of electrochemical interference, the background electrode disposed on a first side of a second insulative substrate, wherein a second side opposite the first side of the first insulative substrate is adjacent to a second side opposite the first side of the second insulative substrate; and an electrical circuit configured to receive the first electrical signal indicative of the amount of glucose and the second electrical signal indicative of the amount of electrochemical interference, wherein the electrical circuit is configured to determine an amount of glucose based on the first signal and the second signal..

Example 28: The glucose monitor system of example 27, wherein the electrical circuit is connected to the electrochemical cell via a first electrical contact in contact with the first surface of the first insulative substrate, wherein the electrical circuit is connected to the electrode via a second electrical contact in contact with a connector configured to contact the first surface of the second insulative substrate and the second electrical contact.

Example 29: The glucose monitor system of any of examples 27 and 28 or any of examples 27 and 28, wherein a drug eluting layer is disposed on at least one of a surface of the background electrode, the first side of the second body proximal to the background electrode, the first side of the second body distal to the background electrode, or the first side of the second body proximal and distal to the background electrode.

Example 30: The glucose monitor system of any one of examples 27 through 29, wherein a surface area of the background electrode is greater than a surface area of the electrochemical cell.

Example 31: The glucose monitor system of any one of examples 27 through 30, wherein the background electrode is disposed at a first position along a length direction of the second body, the length direction perpendicular to the first and second directions, wherein the electrochemical cell is disposed at the first position along the first body.

Example 32: The glucose monitor system of any one of examples 27 through 31, wherein the background electrode comprises a glucose limiting membrane disposed over the background electrode surface.

Example 33: The glucose monitor system of any one of examples 27 through 32, wherein a drug eluting layer is disposed on a second side of the second body opposite the first side.

Example 34: The glucose monitor system of example 33, wherein an insulator is disposed over a portion of a surface of the background electrode.

Example 35: The glucose monitor system of any of examples 33 and 34, wherein the drug eluting layer is disposed on the first side of the second body and over a surface of the background electrode.

Example 36: The glucose monitor system of any one of examples 27 through 35, wherein the background electrode is further disposed on a second side of the second body.

Example 37: The glucose monitor system of example 36, wherein an insulator is disposed over a portion of a surface of the background electrode on the second side of the second body.

Example 38: The glucose monitor system of any one of examples 27 through 37, wherein an insulator is disposed over a first portion of a surface of the background electrode, wherein a second portion of the surface of the background electrode is coplanar with the insulator.

Example 39: The glucose monitor system of any one of examples 27 through 38, wherein an insulator is disposed over a first portion of a surface of the background electrode, wherein the drug eluting layer is disposed over the insulator at the first portion and over a second portion of the surface of the background electrode, wherein the thickness of the drug eluting layer is the same at the first and second portions.

Example 40: The glucose monitor system of any one of examples 27 through 39, wherein the background electrode is proximal the electrochemical cell.

Example 41: The glucose monitor system of example 40, wherein a surface area of the background electrode comprises an area that is from 20% to 50% a surface area of the electrochemical cell.

Example 42: The glucose monitor system of any one of examples 27 through 41, wherein the background electrode is between the counter electrode and the working electrode.

Example 43: The glucose monitor system monitor system of any one of examples 27 through 42, wherein the electrochemical cell is disposed on a first side of a body, wherein the background electrode is disposed on a second side of the body opposite the first side.

Example 44: The glucose monitor system of example 43, wherein the electrochemical cell comprises a glucose limiting membrane, wherein the background electrode comprises the glucose limiting membrane.

Example 45: The glucose monitor system of any one of examples 27 through 44, wherein the electrochemical interference is caused by acetaminophen.

Example 46: The glucose monitor system of any one of examples 27 through 45, wherein the surface of the electrode comprises an electrocatalytic material.

Example 47: The glucose monitor system of example 46, wherein the electrocatalytic material is at least one of gold or platinum.

Example 48: The glucose monitor system of any one of examples 27 through 47, wherein the drug eluting layer comprises at least one of dexamethasone or dexacetate.

Example 49: A method includes: receiving, from an electrochemical cell, a first signal indicative of an amount of glucose in a fluid of a person; receiving, from a background electrode configured to not catalyze a reaction with glucose and positioned proximate the electrochemical cell, a second signal indicative of an amount of electrochemical interference; and determining a glucose level of the fluid of the person based on the first and second signals.

Example 50: The method of any of examples 24 through 49, wherein the electrochemical cell is disposed on a first side of a first body, wherein the background electrode is disposed on a first side of a second body.

The techniques described in this disclosure may be implemented, at least in part, in hardware, software, firmware or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable medium may cause a programmable processor, or other processor, to perform the method, e.g., when the instructions are executed. Computer-readable media may include non-transitory computer-readable storage media and transient communication media. Computer readable storage media, which is tangible and non-transitory, may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer-readable storage media. It should be understood that the term "computer-readable storage media" refers to physical storage media, and not signals, carrier waves, or other transient media.

Various examples have been described. These and other examples are within the scope of the following claims.

Further disclosed herein is the subject-matter of the following clauses:
1. A device comprising:
   an electrochemical cell configured to generate a first electrical signal indicative of an amount of glucose in a fluid of a person, the electrochemical cell comprising a working electrode, a counter electrode, and a reference electrode; and
   a background electrode configured to not catalyze a reaction with glucose and is configured to generate a second electrical signal indicative of an amount of electrochemical interference proximate the electrochemical cell.
2. The device of clause 1, wherein the electrochemical cell is disposed on a first side of a first body, wherein the background electrode is disposed on a first side of a second body separate from the first body.
3. The device of clause 1 or 2, wherein the first side of the first body faces a first direction, wherein the first side of the second body faces a second direction opposite the first direction, wherein a second side of the first body opposite the first side of the first body is adjacent a second side of the second body, the second side of the second body opposite the first side of the second body.
4. The device of clause 3 or of any of the clauses 1 to 3, wherein a drug eluting layer is disposed on the first side of the second body.
5. The device of clause 4 or of any of the clauses 1 to 4, wherein the drug eluting layer is disposed on the first side of the second body proximal to the background electrode.
6. The device of clause 4 or of any of the clauses 1 to 5, wherein the drug eluting layer is disposed on the first side of the second body distal to the background electrode.
7. The device of clause 4 or of any of the clauses 1 to 6, wherein the drug eluting layer is disposed on the first side of the second body proximal and distal to the background electrode.
8. The device of clause 3 or of any of the clauses 1 to 7, wherein a surface area of the background electrode is greater than a surface area of the electrochemical cell.
9. The device of clause 3 or of any of the clauses 1 to 8, wherein the background electrode is disposed at a first position along a length direction of the second body, the length direction perpendicular to the first and second directions, wherein the electrochemical cell is disposed at the first position along the first body.
10. The device of clause 4 or of any of the clauses 1 to 9, wherein the drug eluting layer is disposed on at least a portion of the surface of the background electrode.
11. The device of clause 4 or of any of the clauses 1 to 10, wherein the background electrode comprises a glucose limiting membrane disposed over the background electrode surface.
12. The device of clause 1 or of any of the clauses 1 to 11, wherein the background electrode is positioned between the counter electrode and the working electrode.
13. The device of clause 1 or of any of the clauses 1 to 12, wherein the electrochemical cell is disposed on a first side of a first body, wherein the background electrode is disposed on a second side of the first body opposite the first side.
14. The device of clause 1 or of any of the clauses 1 to 13, wherein the electrochemical interference is caused by acetaminophen.
15. The device of clause 1 or of any of the clauses 1 to 14, wherein the surface of the electrode comprises an electrocatalytic material, wherein the electrocatalytic material is at least one of gold or platinum.
16. A glucose monitor system comprising:
   an electrochemical cell configured to generate a first electrical signal indicative of an amount of glucose in a fluid of a person, the electrochemical cell disposed on a first side of a first insulative substrate;
   a background electrode configured to not catalyze a reaction with glucose and is configured to generate a second electrical signal indicative of an amount of electrochemical interference, the background electrode disposed on a first side of a second insulative substrate, wherein a second side opposite the first side of the first insulative substrate is adjacent to a second side opposite the first side of the second insulative substrate; and
   an electrical circuit configured to receive the first electrical signal indicative of the amount of glucose and the second electrical signal indicative of the amount of electrochemical interference, wherein the electrical circuit is configured to determine an amount of glucose based on the first signal and the second signal.
17. The glucose monitor system of clause 16, wherein the electrical circuit is connected to the electrochemical cell via a first electrical contact in contact with the first surface of the first insulative substrate, wherein the electrical circuit is connected to the electrode via a second electrical contact in contact with a connector configured to contact the first surface of the second insulative substrate and the second electrical contact.
18. The glucose monitor system of clause 16 or 17, wherein a drug eluting layer is disposed on at least one of a surface of the background electrode, the first side of the second body proximal to the background electrode, the first side of the second body distal to the background electrode, or the first side of the second body proximal and distal to the background electrode.
19. The glucose monitor system of clause 16 or of any of the clauses 16 to 18, wherein a surface area of the background electrode is greater than a surface area of the electrochemical cell.
20. A method comprising:
   receiving, from an electrochemical cell, a first signal indicative of an amount of glucose in a fluid of a person;
   receiving, from a background electrode configured to not catalyze a reaction with glucose and positioned proximate the electrochemical cell, a second signal indicative of an amount of electrochemical interference; and
   determining a glucose level of the fluid of the person based on the first and second signals.

## Claims

1. A device comprising:
an electrochemical cell configured to generate a first electrical signal indicative of an amount of glucose in a fluid of a person, the electrochemical cell comprising a working electrode, a counter electrode, and a reference electrode; and
a background electrode configured to not catalyze a reaction with glucose and is configured to generate a second electrical signal indicative of an amount of electrochemical interference proximate the electrochemical cell.

2. The device of claim 1, wherein the electrochemical cell is disposed on a first side of a first body, wherein the background electrode is disposed on a first side of a second body separate from the first body.

3. The device of claim 2, wherein the first side of the first body faces a first direction, wherein the first side of the second body faces a second direction opposite the first direction, wherein a second side of the first body opposite the first side of the first body is adjacent a second side of the second body, the second side of the second body opposite the first side of the second body.

4. The device of claim 2 or 3, wherein a drug eluting layer is disposed on the first side of the second body.

5. The device of claim 4, wherein the drug eluting layer is disposed on the first side of the second body proximal to the background electrode, or
wherein the drug eluting layer is disposed on the first side of the second body distal to the background electrode, or
wherein the drug eluting layer is disposed on the first side of the second body proximal and distal to the background electrode.

6. The device of one of the claims 1 to 5, wherein a surface area of the background electrode is greater than a surface area of the electrochemical cell.

7. The device of one of the claims 3 to 6, wherein the background electrode is disposed at a first position along a length direction of the second body, the length direction perpendicular to the first and second directions, wherein the electrochemical cell is disposed at the first position along the first body.

8. The device of one of the claims 4 to 7, wherein the drug eluting layer is disposed on at least a portion of the surface of the background electrode.

9. The device of one of the claims 1 to 8, wherein the background electrode comprises a glucose limiting membrane disposed over the background electrode surface.

10. The device of one of the claims 1 to 9, wherein the background electrode is positioned between the counter electrode and the working electrode, and/or
wherein the electrochemical cell is disposed on a first side of a first body, wherein the background electrode is disposed on a second side of the first body opposite the first side.

11. The device of one of the claims 1 to 10, wherein the electrochemical interference is caused by acetaminophen, and/or
wherein the surface of the electrode comprises an electrocatalytic material, wherein the electrocatalytic material is at least one of gold or platinum.

12. A glucose monitor system comprising:
an electrochemical cell configured to generate a first electrical signal indicative of an amount of glucose in a fluid of a person, the electrochemical cell disposed on a first side of a first insulative substrate;
a background electrode configured to not catalyze a reaction with glucose and is configured to generate a second electrical signal indicative of an amount of electrochemical interference, the background electrode disposed on a first side of a second insulative substrate, wherein a second side opposite the first side of the first insulative substrate is adjacent to a second side opposite the first side of the second insulative substrate; and
an electrical circuit configured to receive the first electrical signal indicative of the amount of glucose and the second electrical signal indicative of the amount of electrochemical interference, wherein the electrical circuit is configured to determine an amount of glucose based on the first signal and the second signal.

13. The glucose monitor system of claim 12, wherein the electrical circuit is connected to the electrochemical cell via a first electrical contact in contact with the first surface of the first insulative substrate, wherein the electrical circuit is connected to the electrode via a second electrical contact in contact with a connector configured to contact the first surface of the second insulative substrate and the second electrical contact.

14. The glucose monitor system of claim 12 or 13, wherein a drug eluting layer is disposed on at least one of a surface of the background electrode, the first side of the second body proximal to the background electrode, the first side of the second body distal to the background electrode, or the first side of the second body proximal and distal to the background electrode, and/or
wherein a surface area of the background electrode is greater than a surface area of the electrochemical cell.

15. A method comprising:
receiving, from an electrochemical cell, a first signal indicative of an amount of glucose in a fluid of a person;
receiving, from a background electrode configured to not catalyze a reaction with glucose and positioned proximate the electrochemical cell, a second signal indicative of an amount of electrochemical interference; and
determining a glucose level of the fluid of the person based on the first and second signals.
